# EUROPEAN PATENT APPLICATION

(11) **EP 2 206 470 A2**
(43) Date of publication of application: **14.07.2010**
(21) Application number: 09075581.0
(22) Date of filing: 21.05.2009
(51) Int. Cl.: A61B 17/86, A61B 17/68

(54) **Systems for the medical treatment of structural tissue**

(30) Priority: 21.05.2008 US 124416; 21.05.2008 US 124446
(71) Applicant: Gooch, Hubert L., Concord, Nc 28027 (NC)
(72) Inventor: Gooch, Hubert L., Concord, Nc 28027 (NC)
(74) Representative: Denison, Christopher Marcus

(57) **Abstract**

The present invention is directed generally to a bio-medical system(s). More specifically, some embodiments may include system(s) used in medical treatment(s). Various embodiments may include medical system(s) that may use an anchoring mechanism (100) and screw (bolt, etc.) arrangement that increases the strength of attachment. Some embodiments may further include, for example, an anchoring mechanism (140) that expands upon insertion of a screw (bolt, etc.) into an opening. Some embodiments may include, for example, a system(s) for improvement of structural tissue (e.g., bone, cartilage) fixation and safety. Further, some embodiments may also include, for example, an anchoring mechanism and screw (bolt, etc.) system that may be utilized in orthopedic procedures. Still some embodiments may further include system(s) providing improved screw fixation and enhanced stabilization in structural tissue or fracture repair. Various embodiments may include multiple anchor mechanisms and anchor mechanism holding means.

## Description

### BACKGROUND

### FIELD OF THE INVENTION

The present invention relates to the field of bio-medical devices and methods, more specifically, a device(s) and method(s) used in medical treatment(s).

### DESCRIPTION OF RELATED ART

There are several systems and methods previously described which are utilized in medical treatments relating to various structural tissues. Some of the more common treatments include the insertion and/or placement of screws and related instrumentation into or onto bone to alleviate or treat orthopedic conditions. There is an unaddressed need, however, for better systems and methods for the treatment of structural tissue in which screw purchase (e.g., gripping, reduced pullout, etc.) may be improved or augmented, damage to the surrounding structural tissue may be minimized, while efficiency and speed of the medical procedure may be improved.

While many areas of medicine and structural tissue anomalies may present difficulties, some particularly difficult areas may involve orthopedic procedures concerning complex or sensitive areas, e.g., the spinal column. When considering the spinal column, in addition to damage of the surrounding tissue of the pedicle wall, once a pedicle or bone screw is inserted, damage or irritation to the spinal cord may be a concern if the cortical pedicle wall becomes breached.

Prior devices have inadequately addressed the problem of safely and quickly improving screw purchase or gripping within structural tissue that may be efficiently implanted during a surgical procedure. This is particularly important during spinal surgery or other operations where blood loss may be a concern. Some exemplary inadequate approaches are provided by the following patents.

The use of pedicle screws and/or bone screws in an orthopedic context is seen in several treatments involving the spinal column. For example, U.S. Patent No. 7,056,321 describes a method of fixing the first and second vertebrae of a patient together by utilizing a cannula (tube), through which a fusion device, fasteners, and fixation brackets or rods are introduced. Completion of the procedure should result in fixing together a first and second vertebrae. U.S. Patent No. 7,087,056 describes a vertebral stabilization assembly for stabilizing vertebrae, which includes a pedicle screw(s), connecting screw(s), and a connecting rod or bar. These procedures and similar procedures are particularly tedious and take more time than would be preferred given a patient's loss of blood during the surgical procedure.

U.S. Patent No. 7,090,675 describes a cable anchoring mechanism in the form of a screw member having an elongated shank that is threaded for substantially its entire length to enable the screw member to be fully sunk into the bone so that no portions project into the surrounding body cavity. U.S. Patent No. 5,084,050 describes an implant for bone reinforcement and for anchoring bone screw, implants, and implant parts. U.S. Patent No. 5,713,905 describes various types of selectively expandable sacral fixation screw-sleeve device. U.S. Patent Application Pub. No. 2007/0038219 describes an anchoring shaft for use in bone tissue that utilizes a plurality of barb elements to anchor the shaft.

Further, U.S. Patent No. 2,381,050 describes a body member that can be implanted into bone with an internal bore through which a threaded rod or bolt may be inserted to cause the body member to expand. U.S. Patent Application Pub. No. 2006/0095040 describes a bone screw for use with a radially expandable sleeve for introduction into a medullary pin. U.S. Patent Pub. No. 2004/0193157 describes a bone anchor that utilizes outwardly advancing tangs or barbs to fix the anchor within bone. U.S. Patent No. 4,640,271 describes a bone screw with a threaded sleeve that is slidable about an unthreaded portion of the bone screw. U.K. Patent Pub. No. GB2084468 describes a stud for anchoring in a hole formed in bone that expands upon insertion of a pin.

However, these devices are inadequate in providing sufficient screw purchase augmentation, ease and speed of implementation, efficiency, and/or safety. For example, inserting a screw into a pedicle channel applies substantial force to the structural tissue surrounding the screw both laterally and radially. Further, the various devices and procedures described above have not addressed difficulties that may arise from performing these procedures on particularly delicate or fragile bones such as those that are osteoporotic. Still further, the introduction of a screw into the pedicle must be done sensitive to the proximity of the pedicle to the spinal cord and nerve roots and the fact that the nerve system is closely adjacent thereto. In these situations, current devices for anchoring and improved screw insertion provide inadequate ways of protecting the spinal cord or nerve roots.

Additionally, an improperly placed pedicle screw or one that has become loose can pose difficulties. The pedicle channel may have become worn over time due to repeated stresses from structural tissue movement that occurs in everyday human body activity. A pedicle screw with a pedicle channel that has become worn will likely not function as optimally as originally intended causing instrumentation to become loose, resulting in pain or discomfort to the patient. A worn pedicle channel and loose pedicle screw or mounting brackets may contribute to further damage of the pedicle channel and possible breach of a pedicle channel or spinal bone. Previously known devices and methods have generally been unable to deal with the difficulties that may result from breaching of the pedicle cortex or bone into the spinal canal.

Thus, current methods do not provide an adequate system by which to utilize a device for augmenting purchase of devices or screws inserted and attached to structural tissue such as bone. The present invention provides a solution to many of the problems associated with inserting a bone or pedicle screw into various types of structural tissue, e.g., a bone or vertebra and the difficulties in dealing with a damaged, worn, or improperly placed bone screw, anchor, or pedicle channel. For example, the present application describes devices or mechanisms and methods, which may be introduced for improving screw purchase, mounting device or bracket purchase, or gripping within structural tissue.

### SUMMARY

The present invention is directed generally to bio-medical device(s) and method(s). More specifically, some embodiments may include device(s) and method(s) used in various medical treatments that relate to repairing, reconstructing, replacing, attaching, connecting or repairing structural tissue such as bones, ligaments, tendons, cartilage, etc.

Various embodiments of the present invention may include various systems and methods that improve screw (bolt, post, etc.) and/or instrumentation attachment and/or fixation in medical treatments, particularly orthopedic treatments. For example, various embodiments of the present invention may include the use of an anchoring mechanism(s) used in conjunction with one or more anchor expansion mechanisms (e.g., shaft(s), screw(s), bolt(s), etc.) for attaching and/or affixing an instrumentation(s), screw(s), rods, plates, bolt(s), etc., to structural tissue, such as bone, cartilage, ligament, tendon, etc.

In some embodiments, the anchoring mechanism(s) may be constructed to have one or more movable and/or malleable portions such as external wall members that may expand upon insertion of a shaft(s), screw(s), bolt(s), etc., to provide increased purchase, gripping, or attachment between a screw, a holding means and/or an anchoring mechanism(s), and structural tissue. In various embodiments, the anchoring mechanism may have one or more slits along its wall(s) that enable various parts of the anchoring mechanism to expand upon insertion of a shaft(s), screw(s), bolt(s), etc. In any case, either or both the inner and outer surfaces and/or walls of the anchoring mechanism(s) may be tapered, straight, threaded, and/or smooth. An opening in the anchor for insertion of expansion mechanisms (e.g., shaft(s), screw(s), bolt(s), etc.) may be of varying dimensions to accommodate a desired diameter or length of screw. A shaft, screw, bolt, etc. may be inserted into, attached or affixed to the anchoring mechanism(s) so as to expand the anchoring mechanism(s) and may be used to attach one or more instrumentations thereto. For example, a plate may be attached to a bone screw and the bone screw may be inserted into, attached or affixed to the anchoring mechanism. Usually, a screw or bolt may be placed into the bone and then the plate or rod is attached to the screw or bolt. In this manner, the external surface size of the anchoring mechanism(s) with an expansion mechanism such as a shaft(s), screw(s), bolt(s), etc., inserted therein may increase purchase gripping or attachment between the structural tissue (e.g., bone) and the shaft(s), screw(s), bolt(s), etc. and/or anchoring mechanism(s). In various embodiments the anchoring mechanism(s) may provide for reduction of micro-motion of a pedicle or bone screw, that may be caused by the movement of the instrumentation or bone, so as to reduce friction and/or structural tissue wear-out, for example, by including a moveable and/or malleable portion to the anchor mechanism. For example, the movable or malleable portions may act as a shock absorber or pivot point cushion so as to reduce wear and tear of the structural tissue to attachment mechanism interface.

In at least one embodiment, the anchoring mechanism(s) may be made to include or be coated with a biocompatible material, for example, allograft, allograft bone, polyetheretherkeetones (PEEK), high molecular weight polyethylene (HMWPE), carbon fiber, and/or any other synthetic material that can be manufactured and implanted into the body etc. In at least one embodiment, the anchoring mechanism(s) may be coated with, for example, hydroxylapatite, bone morphogenetic protein, or other such materials, etc., that may promote or induce the formation of structural tissue including, for example, bone, cartilage, etc. In at least one embodiment of the present invention, the external walls of the anchoring mechanism(s) may be threaded, ribbed, studded, or otherwise textured to allow the anchoring mechanism(s) to be more securely or easily placed into a previously formed, drilled, or tapped pedicle, long bone, etc and to increase attachment strength (purchase) between the structural tissue and anchoring mechanism(s).

In at least one embodiment, the anchoring mechanism(s) may be used in conjunction with one or more screws (expansion rods, bolts, plates, rods, tethers, and/or cords, etc.) for fixation during medical procedures. In at least one embodiment, the anchoring mechanism(s) may be placed into a previously tapped hole in structural tissue, for example, bone, ligament, cartilage, etc. In this manner, the anchoring mechanism(s) may eliminate the need to use a screw (bolt, etc.) of larger dimensions for previously used, drilled, or tapped holes that have become worn, larger, and/or loose over time. The anchoring mechanism may also be a better fit to odd or unevenly shaped holes that may develop from prior instrumentation movement. A screw (bolt, etc.) may be introduced into the anchoring mechanism(s) before, during, or after the anchoring mechanism's insertion into the structural tissue. A means for holding the anchoring mechanism(s) may be provided to improve placement and maintain positioning of the anchoring mechanism(s) during insertion and/or assembly of the one or more screws (bolts, etc.) for the anchoring mechanism(s).

At least one embodiment of the anchoring mechanism(s) may include a split tip, which may be split once or multiple times, that may expand upon insertion of a screw (bolt, etc.). In at least one embodiment of the anchoring mechanism(s), the anchoring mechanism(s) may include an at least partially split side which may allow expansion of the anchoring mechanism(s) upon insertion of a screw (bolt, etc.). At least one embodiment of the anchoring mechanism(s) may include a ribbed, studded, textured, etc., outer surface that may aid in screw purchase or gripping.

In some cases, the pedicle channel may have become worn such that the angle, inclination, and/or direction of the original channel has not been maintained. In at least one embodiment, the anchoring mechanism(s) may be inserted into such an obliquely started, worn, or oblong channel, and the screw (bolt, etc.) may be inserted into the anchoring mechanism(s) at an angle, for example an oblique angle, relative to the direction of the length-wise position of the anchor mechanism, so as to increase purchase or attachment in the channel, e.g., a pedicle channel, while restoring the originally intended angle of the screw (bolt, etc.). In various embodiments, an angled hole may be placed or manufactured into the anchoring mechanism prior to the screw being inserted.

In at least one embodiment, an anchoring mechanism(s) may be used for insertion into another anchoring mechanism(s). The anchoring mechanism(s) may be inserted into another anchoring mechanism(s) at a direct angle or oblique angle or both. The anchoring mechanism(s) may be made of a malleable material which may allow another anchoring mechanism(s) to be easily inserted by, for example, drilling, punching, or forcing an off-center or angled hole. Once an anchoring mechanism(s) may be inserted inside of another anchoring mechanism(s), a screw (bolt, etc.) may be inserted into one or more of the anchoring mechanism(s), which may increase screw purchase or attachment at an opening in the another anchoring mechanism and may form a "t" or hook-like anchoring mechanism.

In at least one embodiment of the present invention, the interior and/or exterior walls of the anchoring mechanism(s) may be smooth. According to at least one embodiment of the present invention, the interior walls of the anchoring mechanism(s) may be threaded to correspond to the threading of a screw (bolt, etc.). In at least one embodiment, placement of the anchoring mechanism(s) may cover any breaching of the pedicle wall and may protect the exposed nerve root or spinal cord. For example, a portion of one exterior side of the anchoring mechanism(s) may be smooth so that it may be oriented in the direction of a nerve of spinal cord in the case that an exterior bone material such as the cortical bone or cortical wall may have been breached.

In at least one embodiment of the anchoring mechanism(s), the anchoring mechanism(s) may be in the shape of a helical coil, which may be inserted into e.g., a bone screw or a pedicle channel. The helical coil may be made of a metal and/or malleable material. The helical coil may be constructed such that insertion of a screw (bolt, etc.) such as a bone or pedicle screw may cause the helical coil to expand, which may increase purchase between the helical coil, the screw, and the surrounding structural tissue. The helical coil may be constructed such that different degrees of tapering of an inserted screw may impact the degree and/or location of expansion of the helical coil which may increase purchase between the helical coil and the surrounding tissue. The screw (bolt, etc.) may be inserted at a direct angle, an oblique angle, or both, according to at least one embodiment.

The helical coils may be constructed of a malleable material and/or contain a biocompatible coating, which may be less likely to breach the walls of the pedicle. In the event that the pedicle wall becomes breached, the helical coil constructed of a malleable material or containing a biocompatible coating will not likely irritate or harm the surrounding nerve tissue adjacent to the point where the pedicle wall has been breached.

In at least one embodiment of the anchoring mechanism(s), the anchoring mechanism may be constructed from a biomaterial such as bone tissue generated, manufactured, and/or grown by, for example, a stem cell based process. The stem cells may be provided from cells of the patient, other people, fetus cells, or synthetically according to various known or developing techniques. The use of such a biogenerated and/or grown material may allow the screw (bolt, etc.) to be secured in the pedicle without some of the typical difficulties associated with introducing foreign materials into the human body.

In at least one embodiment of the anchoring mechanism(s), the anchoring mechanism(s) may include a means for holding or a holder or mechanism(s) to grip the anchoring mechanism(s). The holder or grip may be utilized so as to minimize torsion against the surrounding structural tissue and/or increase placement accuracy. Various embodiments of the anchoring mechanism(s) may include additional structures on the outside of the anchoring mechanism(s) to counter the torsion applied by inserting a screw (bolt, etc.) by, for example, increasing the friction between the anchoring mechanism and structural tissue. For example, the anchoring mechanism(s) may include de-rotational "fins" or studs that may be impacted into structural material, e.g., bone. Further, a clamp means may be used to hold the anchor by friction and/or insertion or inclusion of studs may be place into de-rotational holes around the top of the anchoring mechanism(s).

At least one embodiment of the anchoring mechanism(s) may include visual markers to allow determination of the rotational and/or angular orientation of the anchoring mechanism during the procedure. At least one embodiment of the anchoring mechanism(s) may incorporate an x-ray opaque design feature to enable visualization and/or manipulation of the anchoring mechanism(s) in vivo utilizing plain x-ray and/or fluoroscopy, etc.

Still further configurations or variations included for various embodiments will be apparent to one skilled in the art based on the study of the following disclosure and the accompanying drawings thereto. The following descriptions of the present invention provided herein are provided as exemplary of the various aspects and embodiments of the invention and are not intended to be limiting on the invention.

### BRIEF DESCRIPTION OF THE DRAWINGS

The utility, objects, features. and advantages of the invention will be readily appreciated and understood from consideration of the following detailed descriptions of the embodiments of this invention, when taken with the accompanying drawings, for which a brief description follows:

Figs. 1A and 1B show exemplary perspective views of an anchoring mechanism(s) in its unexpanded and expanded forms, respectively, and screw (Fig. 1B), according to various embodiments;

Figs. 2A and 2B show exemplary perspective views of an anchoring mechanism(s) in its unexpanded and expanded forms, respectively, where the inner walls of at least a portion of the anchoring mechanism(s) are tapered, according to at least one embodiment;

Fig. 3A and 3B show exemplary perspective views of an anchoring mechanism(s) in its unexpanded and expanded forms, respectively, where the outer walls of at least a portion of the anchoring mechanism(s) are tapered and at least a portion of the outer walls of the anchoring mechanism(s) are threaded;

Fig. 4 is an exemplary top view of an anchoring mechanism(s) in its expanded form, according to at least one embodiment;

Fig. 5 is an exemplary perspective view of an unexpanded anchoring mechanism(s) that is partially threaded and partially smooth, according to at least one embodiment;

Fig. 6 is an exemplary perspective view of an unexpanded anchoring mechanism(s) with a ribbed external surface, according to at least one embodiment;

Fig. 7 is an exemplary perspective view of an unexpanded anchoring mechanism(s) with a studded external surface, according to at least one embodiment;

Fig. 8 is an exemplary perspective view of an unexpanded anchoring mechanism(s) containing visual markers and/or x-ray opaque markers to determine rotational orientation, according to at least one embodiment;

Fig. 9 is an exemplary perspective view of an unexpanded anchoring mechanism(s) with a split tip, according to at least one embodiment;

Fig. 10 is an exemplary perspective view of an unexpanded anchoring mechanism(s) with a split side, according to at least one embodiment;

Fig. 11 is an exemplary top view of a vertebra depicting a screw (bolt, etc.), a pedicle channel, and an anchoring mechanism(s), according to at least one embodiment;

Fig. 12 is an exemplary side view of a vertebra depicting a pedicle channel, a screw (bolt, etc.), and an expanded anchoring mechanism(s), according to at least one embodiment;

Fig. 13 is an exemplary posterior view of a vertebra depicting a pedicle channel, an anchoring mechanism(s), and an opening in the anchoring mechanism(s) for a screw (bolt, etc.), according to at least one embodiment;

Fig. 14 is an exemplary cross-sectional view of a long bone with an inserted anchoring mechanism(s), with a portion of the lateral length expanded, according to at least one embodiment;

Fig. 15 is an exemplary cross-sectional view of a long bone with an inserted anchoring mechanism(s), with most of the lateral length expanded, according to at least one embodiment;

Figs. 16A and 16B show exemplary top views of a vertebra depicting a worn pedicle channel with an anchoring mechanism(s) inserted and expanded to aid in improving screw purchase, according to at least one embodiment;

Fig. 17 is an exemplary perspective view of an anchoring mechanism(s) including a screw (bolt, etc.) that allows angled insertion of the screw (bolt, etc.) into the anchor mechanism, according to at least one embodiment;

Fig. 18 is an exemplary perspective view of an anchoring mechanism(s) and a screw (bolt, etc.) that may be used for allowing angled insertion of the screw (bolt, etc.) where the screw (bolt, etc.) has been inserted into the anchoring mechanism(s) at a side location, according to at least one embodiment;

Fig. 19 is an exemplary perspective view of multiple anchoring mechanism(s) that allows angled insertion of one anchoring mechanism(s) into another, and the subsequent insertion of a screw, according to at least one embodiment;

Fig. 20 is an exemplary top view of a vertebra depicting a worn pedicle channel with an anchoring mechanism(s) inserted with insertion of the screw (bolt, etc.) at an angle relative to the lateral length of the anchoring mechanism, so as to aid in redirecting screw purchase, according to at least one embodiment;

Fig. 21 is an exemplary top view of a vertebra depicting an anchoring mechanism(s) inserted into another anchoring mechanism(s) via an angled insertion of the anchor to aid in redirecting screw purchase, according to at least one embodiment;

Figs. 22A, 22B, and 22C show an exemplary top view of a vertebra depicting a worn pedicle channel. Fig. 22A depicts a pedicle channel that is worn more heavily near the opening of the pedicle channel. Fig. 22B depicts a pedicle channel that is worn more heavily away from the opening of the pedicle channel. Fig. 22C depicts a pedicle channel that is worn fairly evenly along the length of the pedicle channel;

Figs. 23A and 23B show exemplary perspective and cross sectional views of an anchoring mechanism(s) in the form of helical coil(s) that may expand upon the insertion of a screw (bolt, etc.) to improve screw purchase, according to at least one embodiment;

Fig. 23C shows exemplary perspective view of an anchoring mechanism(s) in the form of a helical coil and a screw that is tapered such that the insertion of the screw causes the helical coil to expand so that an outer surface(s) becomes larger and more even, according to at least one embodiment;

Fig. 24 is an exemplary top view of a vertebra depicting a worn pedicle channel where the wall of the pedicle has been breached and an anchoring mechanism(s) with a smooth portion or portion covered with a protective coating has been inserted, according to at least one embodiment;

Fig. 25 is an exemplary perspective view of an anchoring mechanism(s) with an integrally formed holder that may be removed, according to at least one embodiment;

Fig. 26 is an exemplary perspective view of an anchoring mechanism(s) and forceps where the outer circumference near the top of the anchoring mechanism(s) is perforated , textured, ribbed, indented, etc. and includes a means for gripping it with the forceps, according to at least one embodiment;

Fig. 27 is an exemplary perspective view of an anchoring mechanism(s), guide, and screw (bolt, etc.) where a portion of the anchoring mechanism(s) is formed to interdigitated with the guide, according to at least one embodiment;

Fig. 28 is an exemplary perspective view of an anchoring mechanism(s) where the top contains members that extend laterally outward, according to at least one embodiment;

Fig. 29 is an exemplary perspective view of an anchoring mechanism(s) where the top contains members that extend laterally outward and extend along the length of the anchoring mechanism(s), according to at least one embodiment;

Fig. 30 is an exemplary block diagram depicting one method of using the present invention, according to at least one embodiment;

Fig. 31 is an exemplary block diagram depicting a second method of using the present invention, according to at least one embodiment; and

Fig. 32 is an exemplary block diagram depicting a third method of using the present invention, according to at least one embodiment.

The embodiments of the invention, as described in the brief descriptions of the figures above, are intended to be illustrative and exemplary of the various embodiments of the invention, and should not be construed as limitations on the scope of the invention.

### DETAILED DESCRIPTION

The present invention is directed generally to bio-medical device(s) and method(s). More specifically, the embodiments may include a device(s) and method(s) used in various medical treatments. For example, various embodiments may include device(s) and method(s) that relate to reconstructing, replacing, attaching, connecting or repairing structural tissue such as bones, ligaments, etc. in living cells or organisms. In various embodiments, the present invention may include one or more anchor(s) mechanisms used to attach or repair structural tissue, for example, bones, etc. The various anchor mechanisms may be of a unique design that increases the purchase of, for example, a screw or a bolt. The various anchor mechanisms may be of a type that includes one or more expandable members. The expandable members may be of various geometries and wall designs for use in various types of structural tissue and opening in the structural tissue. In various embodiments, the anchoring mechanism may be tapers have a textured or roughened outer surface that may be, for example, threads, cylindrical ribs, nubs or bumps, etc., that improve purchase and/or insertion of the anchor mechanism into structural tissue. In various embodiment, the anchoring mechanism may include one or more smooth sides and one or more roughened sides, so that the smooth side may be directed to an area that may be exposed to tissue that would be sensitive to texturing, e.g., nerve tissue. In various embodiments, the anchor mechanism may include position markers and/or orientation designators to assist in determining the orientation of the anchor mechanism within the structural tissue to ensure proper desired alignment.

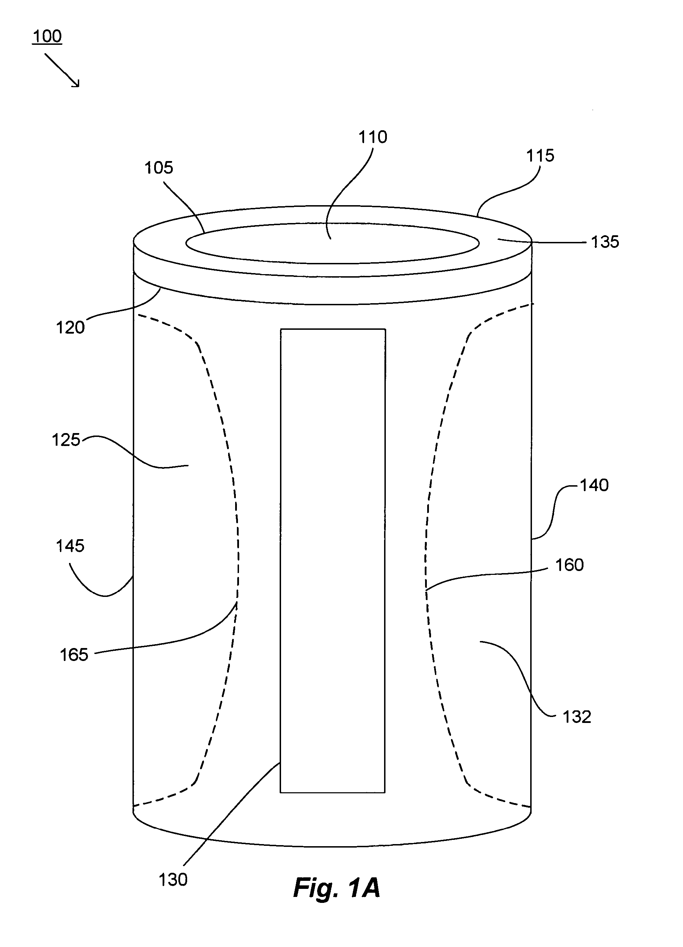

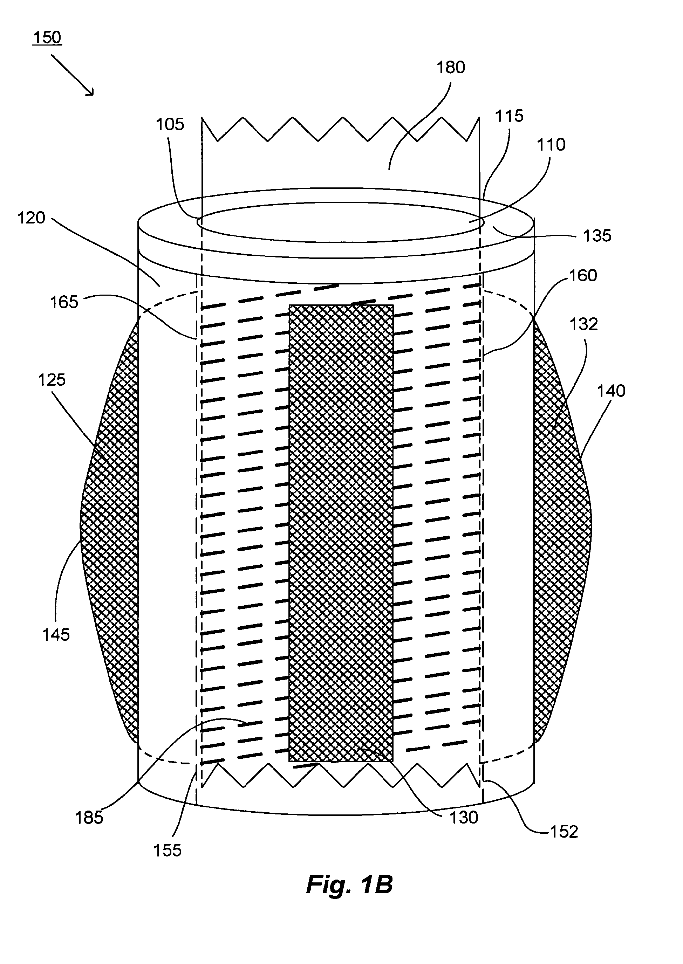

In various embodiments, the anchoring mechanism may include a split tip and/or a split side that has one or more holes formed along its axial length and small attachment sections holding one portion of the anchor mechanism side(s) to another of the anchor mechanism side(s). The anchor mechanism may be made of various materials or coated with various substances to improve the adhesion or purchase between the anchor mechanism and the structural tissue and/or to help the structural tissue grow. The anchor mechanism may be placed at an angle along it lateral axis relative to the lateral axis of the screw or a bolt so as to make a more secure purchase. Multiple anchor mechanisms may also be used at angles to each other's lateral axis and a screw or bolt may be entered into at least one of the anchor mechanisms, so as to make a more secure purchase. The anchor mechanism may be in the shape of a coil that will expand when a screw or bolt is place or screwed into it. For example, the coil may be a helical coil, a coil that is thicker at one end than the other end, or a coil that may expand when used with a tapered screw or bolt. The anchor mechanism may include one or more means for holding it firmly in place while a screw or bolt is inserted and/or screwed into the anchor mechanism. The means for holding may be integrally formed as part of the anchor mechanism, require a special designed tool, and/or be removable attached to the anchoring mechanism. Various methods of using the various anchor mechanisms are also provided. One skilled in the art would recognize that although various embodiments described herein include one or more of these unique features may be combined in numerous combinations together to provide variations that although not shown in the exemplary embodiments described herein, are also covered by the present invention. Some of the various features of the present invention are shown in the exemplary embodiments found in Figs. 1-32 and described below.

Referring to FIG. 1A, a perspective view of one exemplary embodiment of an anchor or anchoring mechanism 100 according to the present invention is provided. In this figure, the anchoring mechanism 100 is unexpanded and is depicted without a screw (bolt, etc.) introduced into an opening 110. In its unexpanded form, the anchoring mechanism 100 may include, for example, a plurality of expanding members, e.g., four expanding members 125, 130, 132, etc., located at quarter portions of, for example, a cylindrical shape. The expanding members 125, 130, 132, etc., may be made of a flexible and/or malleable material, for example, a petroleum based product and/or a material that is proven to be compatable with living organism and/or cells that may be approved for human implantation. In this embodiment, the anchoring mechanism 100 may have an outer surface, e.g., 140, 145, that may be approximately straight and parallel with each other along the lateral length of the anchoring mechanism. Further, the expanding members 125, 130, 132, etc. may have inside surface, e.g., 160 and 165 (i.e., dotted lines showing hidden feature(s)) that may be larger near the midpoint of their lateral length so as to be, for example, curved or bowed inward toward the middle of the cylinder and extend to a width so that they are pushed outward during insertion of a screw or bolt into the opening and through the cylindrical shaped anchor mechanism 100.

The anchoring mechanism 100 may be constructed in the shape of a cylinder and may include an outer surface 115 and an inner surface 105, each of which may include biocompatible material(s), for example, allograft, allograft bone, polyetheretherkeetones (PEEK), high molecular weight polyethylene (HMWPE), carbon fiber, and/or any other synthetic material that can be manufactured and implanted into the body, etc. The anchoring mechanism 100 may be constructed to include a metal and/or malleable material. The outer surface 115 of the anchoring mechanism may be made of, or coated with, hydroxyl apatite, bone morphogenetic protein, etc., for improved structural tissue adhesion and/or growth once the anchoring mechanism is inserted into a structural tissue. The inner surface 105 of the anchoring mechanism 100 may be constructed of a metal and/or malleable material to allow, for example, a screw that is inserted into the opening 110 to self-tap into it. An opening 110 for insertion of a screw (bolt, etc.) may be of varying dimensions to accommodate a desired diameter or length of screw. The top rim 135 of the anchoring mechanism 100 may be of varying dimensions to accommodate the opening 110 of the anchoring mechanism 100 and the outer diameter needed to properly fill a hole formed or worn into structural tissue to which a repair is needed or instrumentation is to be attached thereto. The top rim ring 135 may be made of a different material than the major body 120 of the anchoring mechanism. For example, the top rim ring 135 may be made of metal and the major body 120 may be made of a hard plastic material, for example, a carbon fiber. In one variation, there may be a bottom ring (not shown) to improve the structural rigidity of the anchoring mechanism 100 and/or for proper lateral alignment of the anchoring mechanism in a channel formed in structural tissue using, for example, x-rays to detect the location and orientation. Of course, some alignment and location checking may be accomplished using only the location and pitch of the top rim ring 135.

Referring now to FIG. 1B, a perspective view of one exemplary embodiment of the anchoring mechanism with a shaft, screw or bolt inserted therein 150, according to the present invention is shown. Upon insertion of the shaft, screw, bolt, etc. 180 (partially shown herein), the expanding wall members 170 may expand in an outward direction along at least a portion of the lateral length of the anchoring mechanism. The lateral inner walls 155 of the anchoring mechanism 150 may be straight or tapered so to accommodate the geometry and/or type of shaft (screw, bolt, etc.) 180 being inserted into the anchoring mechanism 100. In this embodiment a screw or bolt shaft 180 having threads 185 is depicted. Once the shaft 180 has been inserted into the anchoring mechanism 100, the outer surface(s) of the shaft will push against the inner walls, e.g., 160 and 165 (i.e., dotted lines showing hidden feature(s)), of the expanding member(s) 125, 130, 132, etc. such that the outside surface of expanding members 125, 130, 132, etc. bow out past the outer surface of the primary structural support walls of the anchor mechanism. Thus, when inserted into a hole or channel within a structural tissue (e.g., bone), the expansion of the expanding (wall) members 125, 130, 132, etc. may increase purchase or gripping between the anchoring mechanism with screw (150) and surrounding structural tissue, beyond the capabilities of only a screw. Further, the expanding (wall) members 125, 130, 132, etc. may be made of a material that absorbs shocks and helps reduce the micro-motion between the shaft and the wall of an opening in a bone into where the anchor 150 is placed. Also, although the expanding members 125, 130, 132, etc. are illustrated in Fig. 1A and 1B as separate pieces to the anchoring mechanism 150, they may be formed as integral pieces of various parts of the anchoring mechanism and may be made of the same material(s), typically malleable or semi-malleable material.

One skilled in the art would recognize that the anchoring mechanism(s) and screw or bolts may be made to come in various length(s) and diameters so as to have multiple sizes available during medical procedures. As such, the anchoring mechanism(s) may include an alignment key or similar structure, so that one anchor mechanism may fit in the screw hole of a larger anchor, so that a smaller diameter screw or bolt may be re-used and the right outer diameter for the pedicle channel can be achieved. Or, two or more anchoring mechanisms may be chained together in series, one abutted or linked to the other(s), and a single pedicle screw or bolt may be inserted into their openings and through their longitudinal axis so as to form a multi-anchoring mechanism structure that may have the same or varying lateral expansion characteristics and length(s) (as may be seen below in various embodiments). Some exemplary pedicle screws sized that may be used in the thoracic and lumbar spine may range from 4 mm to 8 mm in diameter with lengths in the range of 35 mm to 60 or 65 mm. Most typically, screws may be in the 40 to 50 mm in length and the anchoring mechanism(s) may be approximately the same length and have a through hole inner diameter in the range of 4 mm to 8 mm. Of course, as noted above, more than one anchoring mechanism(s) may be used in tandem so as to achieve a variations of lengths or inner diameter.

The embodiment depicted in FIGS. 1A and 1B may be used generally in several procedures including, but not limited to, spinal reconstruction surgery, spinal reworking surgery, and/or long bone reconstruction. One suggested method of utilizing the current invention may involve creating a channel in structural tissue, inserting the anchoring mechanism into the structural tissue channel, and attaching the appropriate instrumentation for spinal fixation during surgery to the anchoring mechanism. Many variations of this process may be utilized, so the procedure(s) described herein is understood to be only exemplary and not as a limitation on the various possible methods for utilizing the embodiment(s).

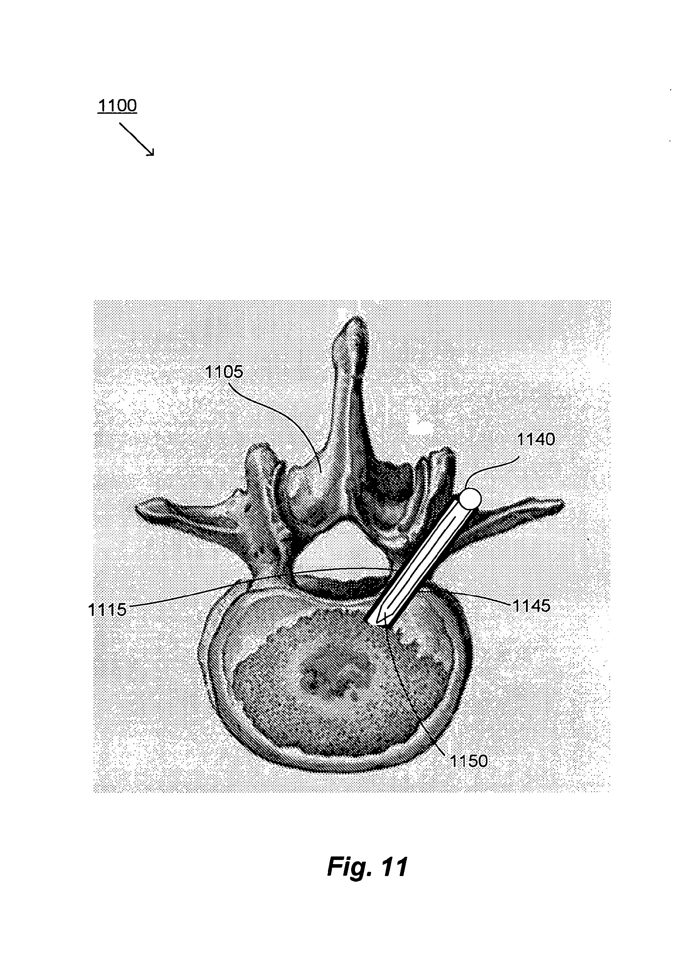

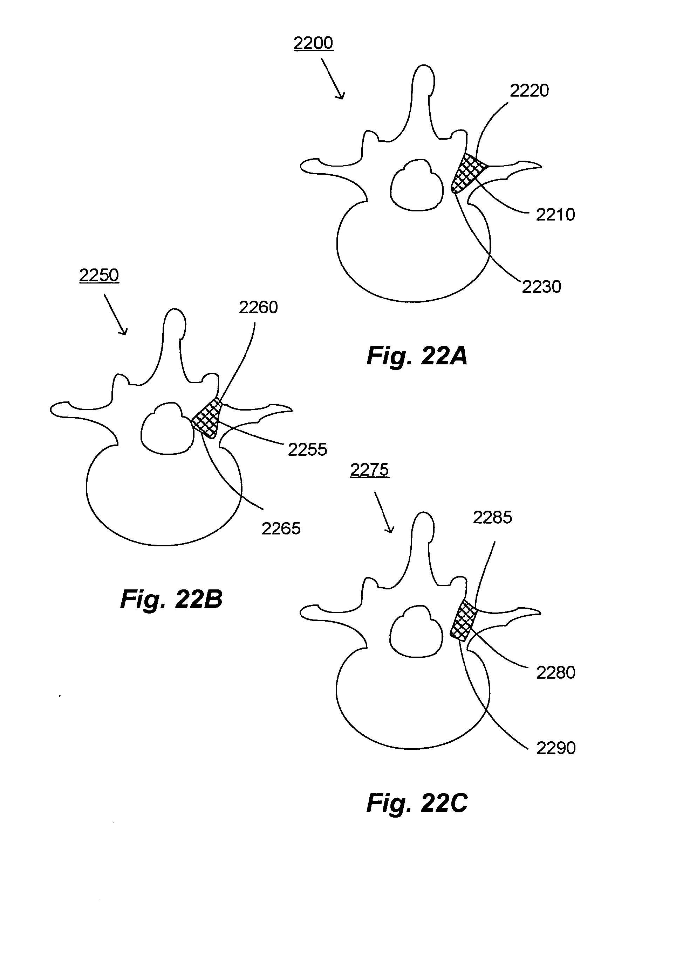

One skilled in the art may utilize this embodiment (and various other embodiment(s) below) by creating a structural tissue channel in, for example, a spinal vertebrae pedicle where a screw, bolt, etc. had previously been secured (see, e.g., Figs. 22A - 22C) or where a shaft, screw, bolt, etc. will be subsequent inserted (see, e.g., Figs. 11-13). The structural tissue channel may be created using any method(s) currently or prospectively known. The channel may be created in a way to facilitate the insertion of an anchoring mechanism as depicted in the embodiment of FIGS. 1A and 1B. The anchoring mechanism may be inserted such that the top opening 110 and rim ring 135 of the anchoring mechanism faces out from the structural tissue upon insertion into the structural tissue channel created in, for example, the pedicle. And, as noted above, the proper orientation or positioning of the anchoring mechanism 150 may be verified using, for example, x-rays, once inserted into the structural tissue channel. A subsequent shaft, screw, bolt, etc. 180 may be inserted into the top opening 110 of the anchoring mechanism 150, which may cause the expandable wall members 125, 130, 132, etc., to expand outward against the walls of the structural channel. The expansion of the expandable (wall) members 125, 130, 132, etc., may thereby exert lateral forces against the walls of the pedicle channel, which may increase purchase, gripping, or friction, between the anchoring mechanism 150 and the surface of the wall of, for example, a pedicle channel. Either before or after the insertion of the shaft, screw, bolt, etc. 180 into the pedicle channel, relevant surgical instrumentation may be attached to the shaft, screw, bolt, etc. to aid in the implementation of spinal reconstruction surgery, spinal reworking surgery, and/or long bone reconstruction.

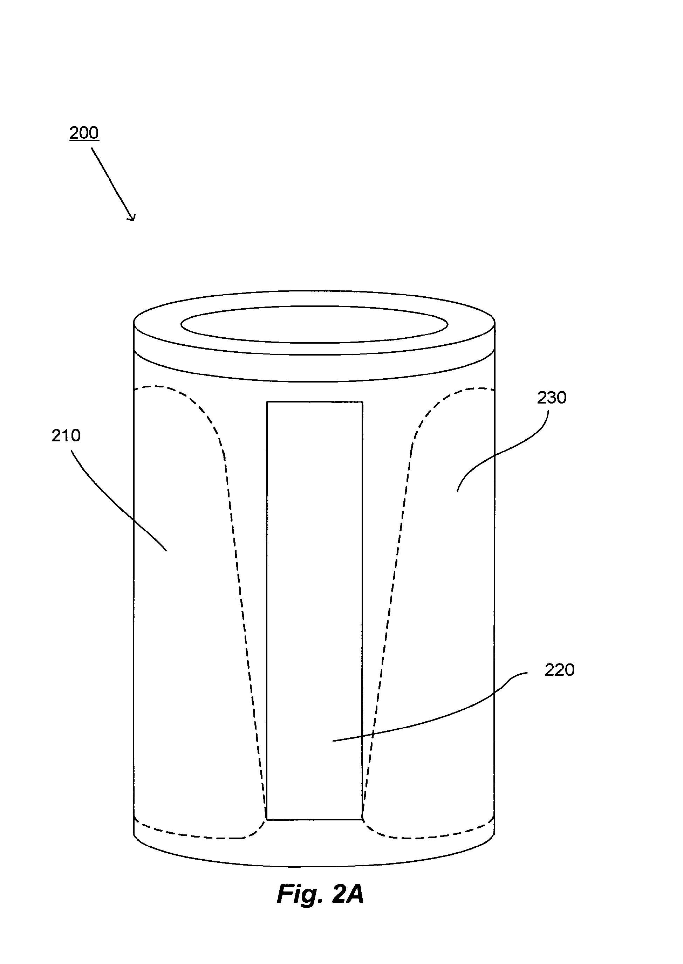

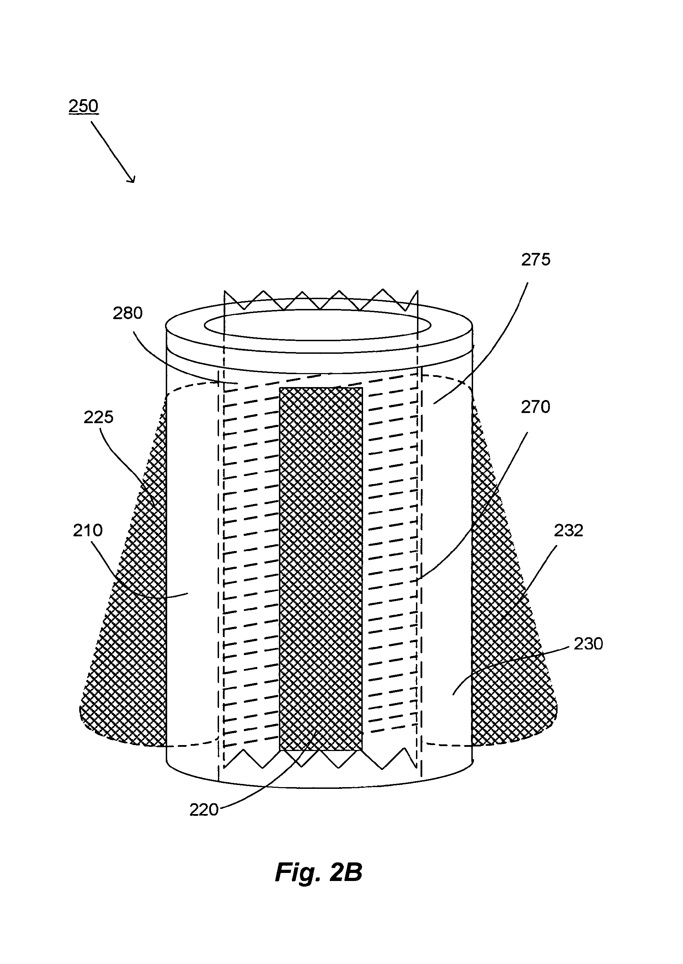

Another exemplary embodiment is shown in Figs. 2A and 2B. Referring to FIG. 2A, a perspective view of one exemplary embodiment of the anchoring mechanism 200 according to the present invention is shown. As shown in this embodiment, the anchoring mechanism 200 is in its unexpanded form. As noted above, the expanding wall members may be made in any of a number of advantageous geometries. In this case, the expanding wall members, e.g., 210, 220, may be constructed such that the inner surface of the expanding members (e.g., walls 210 and 230, having dotted lines showing hidden feature(s) and an outer surface that may be coincident with the outermost surface of the anchor mechanism cylindrical shape primary walls) taper inward along the lateral length of the anchoring mechanism 200 so that when expanded by insertion of a shaft, screw, bolt, etc., they are more vertically extended from the outer surface of the cylinder at, for example, the far end of the cylindrically shaped anchor mechanism. Of course, the expanding members may be inverted to that they are thicker closer to the top opening of the anchor mechanism.

Referring now to FIG. 2B, a perspective view of one exemplary embodiment of the anchoring mechanism 250 including a portion of a rod, screw, bolt, etc., (broken off at each end) is shown. A screw (bolt, rod, etc.) 280 of varying degrees of taper or non-taper may be selected for insertion into the anchoring mechanism such that the degree of taper of the screw (bolt, rod, etc.) may, or may not, directly affect the range of expansion of the expanding wall members, e.g., 210 and 220, to beyond the primary structural walls of the outer cylinder, to e.g., 225 and 232. Due to the interaction between the outermost surface of the screw (bolt, etc.) 270 and the taper of the inner walls 275 of the expanding members 225 and 232, the anchoring mechanism 250 will result in a wider size at the far later length than the top of the anchoring mechanism. Further, if a tapered screw (bolt, etc.) 260 is used it may cause the expanding wall members 225, 232, etc., to expand less; and the less tapered the screw (bolt, etc.) 260 the more the expanding wall members 210, 220, 230, etc., will expand. As such, the selection of various sizes or angle of tapered screw 280 with one or more tapered expanded portions, e.g., 225 and 232, of expandable wall members 210 and 230, can result in a varying possible width anchoring mechanism 250 that may be "right sized" to a desired or resulting hole or channel in structural tissue.

The embodiment depicted in FIGS. 2A and 2B may be used in several procedures including, but not limited to, spinal reconstruction surgery, spinal reworking surgery, and long bone reconstruction. The use of the embodiment is similar to that of the embodiment in FIGS. 1A and 1B. One distinction of the embodiment depicted in FIG. 2A and 2B involves the tapering of one or more of the expanding wall members 210, 220, 230, etc. Further, if a second end ring were included to this embodiment the anchoring mechanism 200, 250 (though not necessary), could facilitate the anchoring mechanism being inverted when inserting into a structural tissue channel so that the larger part of the expanded portions, e.g., 225 and 232, of expandable members 210 and 232, respectively, may work to increase the pull-out strength of the attached screw, bolt, rod, etc., more at the outer portion rather than at the inner portion of a structural tissue channel. Further, if the structural tissue channel into which the anchor mechanism 250 is to be placed also has a taper (see, for example, Figs. 22A and 22b), than the invertible anchor mechanism 250 may be useful in putting a wider portion (when expanded) of the anchor mechanism in the wider area of the structural tissue channel and a narrower portion (when expanded) in the narrower area of the structural tissue channel (when expanded), regardless of which end of the structural tissue channel is wider. As such, the anchoring mechanism 200, 250 has a right sizing aspect to it that may be designed to be more versatile.

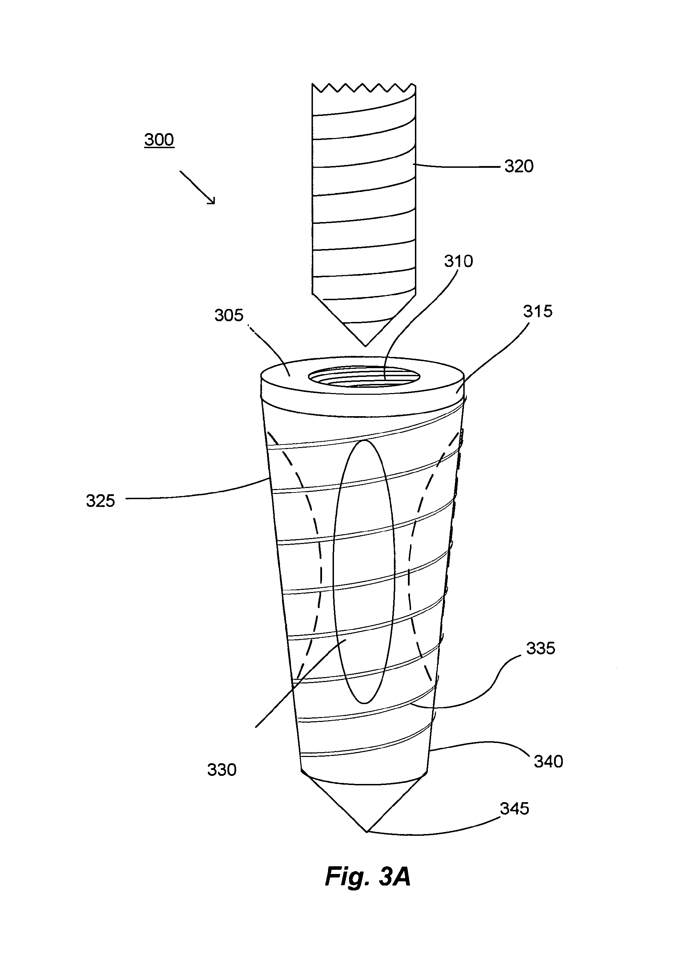

Referring now to FIG. 3A, a perspective view of another exemplary embodiment of the anchoring mechanism 300 according to the present invention is provided. The outer surface 340 of the anchoring mechanism 300 may be tapered in order to aid in gripping and/or insertion of the anchoring mechanism 300 into, for example, structural tissue. The outer side surface 315 of the rim 305 of the anchoring mechanism 300 may be straight or tapered. The rim 305 of the anchoring mechanism 300 may be of varying dimensions in order to accommodate varying dimensions of the opening 310 of the anchoring mechanism 300 and the size of tissue channel the anchor mechanism is to be inserted into. The tip 345 of the anchoring mechanism 300 may be tapered to a point to assist in insertion into the structural tissue channel. The outer surface 340 and the inner surface 310 of the anchoring mechanism may contain threads 335 in order to aid in gripping and/or insertion of the anchoring mechanism 300 into, for example, structural tissue. The inside threads may help facilitate ease of installation of the screw (bolt, etc.) 320 into the opening in the anchor mechanism 300. All or a portion of the outside surface may include threads 325. The outside threads 335 may, but need not be, included on the outer surface of the expanding members 325, 330, etc. In any case, use of thread 335 on the outside surface of the anchoring mechanism 300 may facilitate it being inserted into the structural tissue by screwing it or turning it into the structural tissue. The thread 335 in conjunction with the tapered lateral length and the expanding members 325, 330, etc., may help to significantly improve screw, bolt, etc. purchase in the structural tissue. According to at least one embodiment of the present invention, insertion of a screw (bolt, etc.) 320 into the opening 310 may cause expansion of the expanding wall members, e.g., 330, 325, etc. of the anchoring mechanism 300 resulting in lateral forces expanding outward against structural tissue, for example, a pedicle wall. The lateral forces may improve pullout strength within, for example, a vertebra, a pedicle, a vertebral body, a long bone, etc.

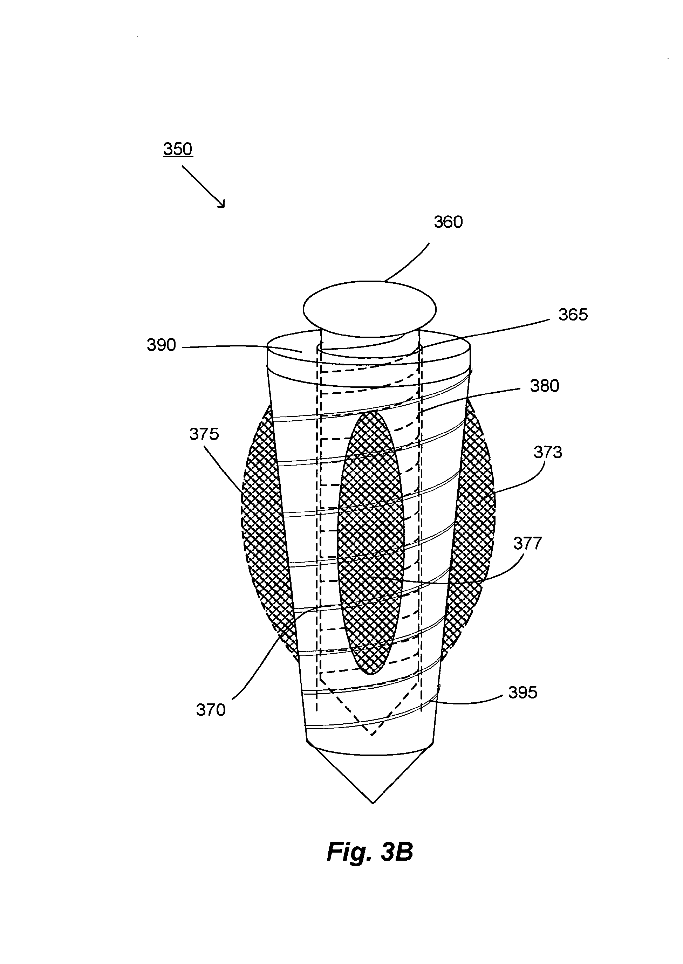

Referring now to FIG. 3B, a perspective view of one exemplary embodiment of the anchoring mechanism 350 according to the present invention is provided. The anchoring mechanism 350 is expanded with the expanding wall members 373, 375, 377, etc. in their expanded form. The anchoring mechanism 350 is depicted with a screw (bolt, etc.) 360 introduced into opening 365. The inner surface 380 of the anchoring mechanism 350 may be threaded to match the threads 370 of the inserted screw (bolt, etc.) 360 or may be smooth. If the inner surface 380 is smooth, it may be composed of a malleable material in order to improve grip when a screw (bolt, etc.) 360 is inserted. All or a portion of the outside surface may include threads 395. As can be seen, this particular embodiment may provide better ease of installation and better improved purchase by utilizing a tapered shape for the anchoring mechanism 350. Although not shown, like discussed above with respect the embodiment shown in Fig. 2A and 2B, use of a tapered screw (bolt, etc.) 360 may advantageously result in a different expanded outer surface contour for the expanding wall members 373, 375, 377, etc. than that shown in Fig. 3B.

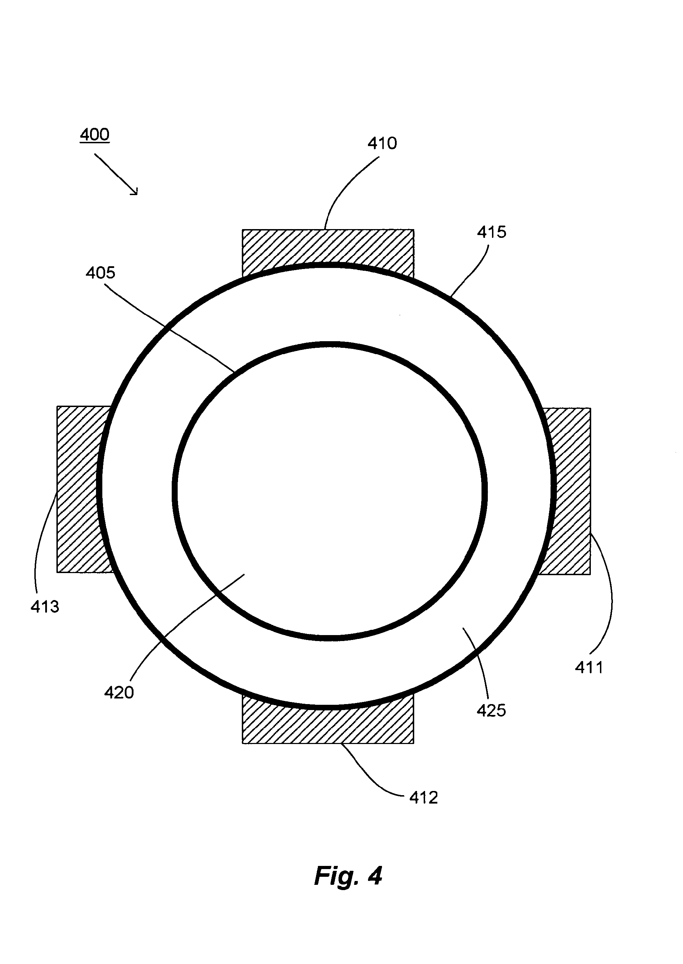

Referring now to FIG. 4, a top view of one exemplary embodiment of the anchoring mechanism 400 according to the present invention is provided. The anchoring mechanism 400 in this example has four expandable members 410, 411, 412, and 413, all in their expanded form. The expanding wall members 410 of the anchoring mechanism have been pushed out from the anchoring mechanism (e.g., like when a shaft, screw, or bolt are inserted into opening 420. Although, the anchoring mechanism 400 is depicted in this figure without a screw (bolt, etc.) inserted into the opening 420 of the anchoring mechanism 400 in order to aid in visualization of the anchoring mechanism. The rim 425 of the anchoring mechanism 400 may or may not be visible from the top upon the insertion of a screw (bolt, etc.) into the opening 420 of the anchoring mechanism 400. As noted above, the screw (bolt, etc.) or other object may typically be necessary in order to engage the expansion of the wall members 410 of the anchoring mechanism 400. Typically, the expansion of the expanding wall members 410 may cause the wall members to expand wider than the outer most primary surface 415 (e.g., made of a rigid structurally solid material) of the anchoring mechanism 400. (Note the situation where the screw is tapered.) The dimensions of the inner walls 405 may vary in diameter to accommodate the varying dimensions needed to fit the screw (bolt, etc.) to be inserted there through.

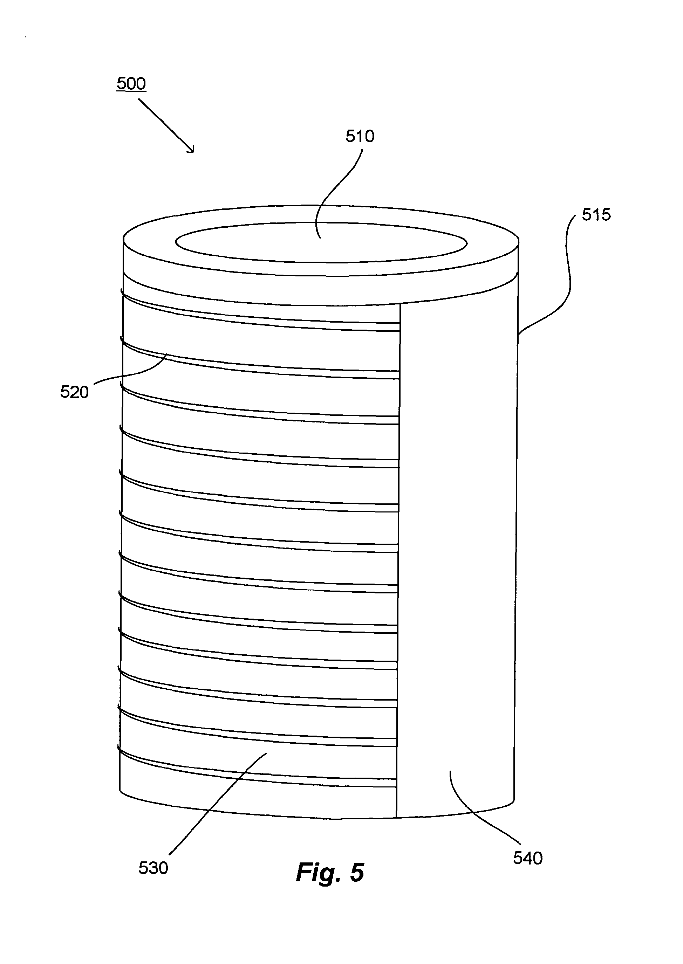

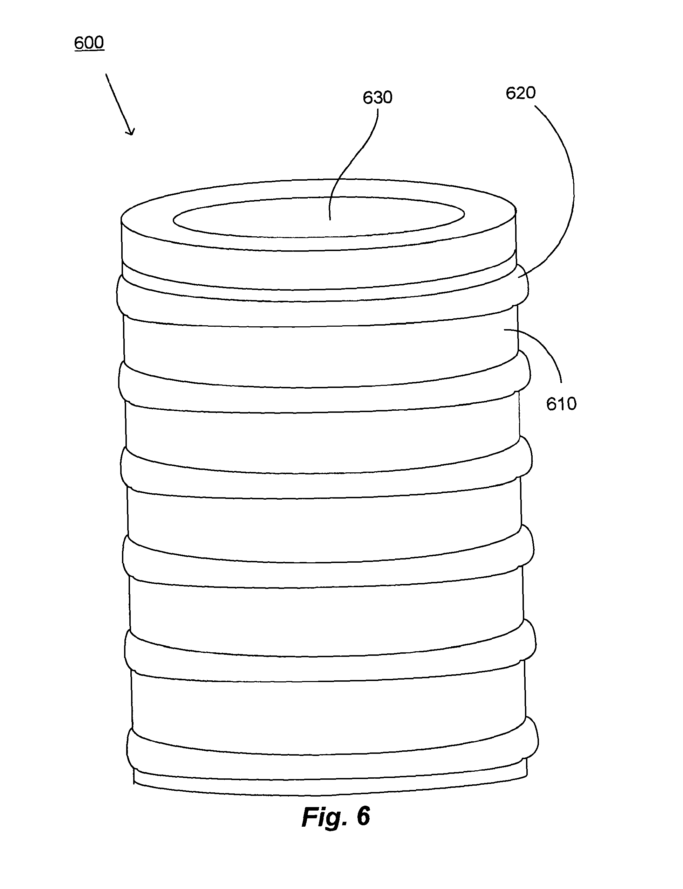

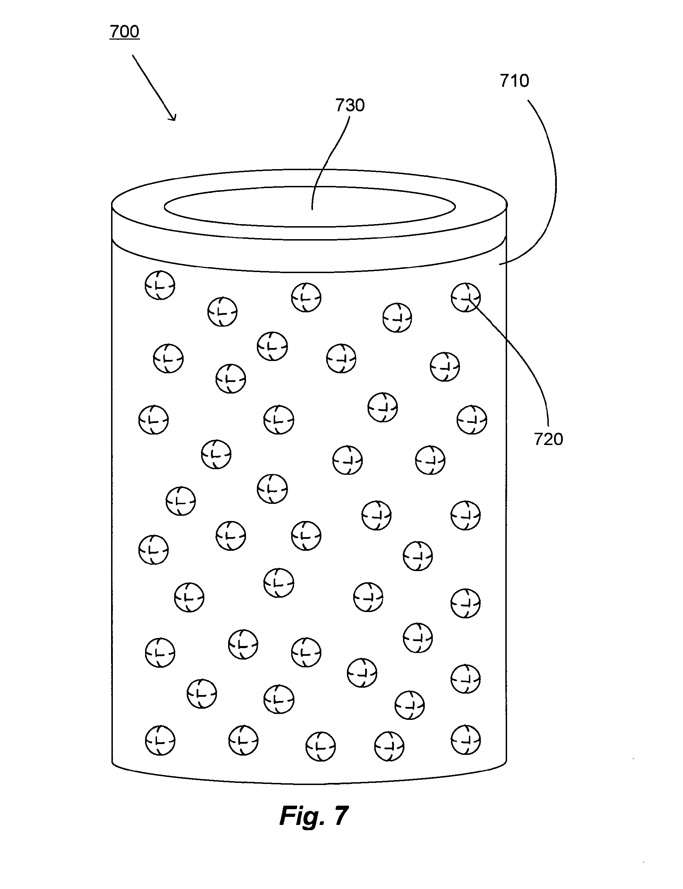

Referring now to Fig. 5, a perspective view is provided of another anchoring mechanism 500, according to at least on embodiment of the present invention. The outer surface 515 of the anchoring mechanism 500 cylindrical shape may include a textured or roughened area 530 and a smooth area 540. These areas may be any portion of the total cylindrical diameter of the anchoring mechanism. Although not show in Fig. 5 (or Figs. 6 and 7) for simplicity, the anchoring mechanism may also include one or more expanding members for increasing gripping or purchase to structural tissue. In any case, the textured or roughened 530 may include, for example, spiral threads 520. The presence of a textured or roughened surface 530 may provide improved grip or purchase to or against the structural tissue where the anchoring mechanism 500 is inserted. The smooth portion 540 may be directed to an area that might not be best served by a textured surface, for example, an area of bone or skin that is thin and may wear away or break easily if interface by a roughen surface, nerve tissue that may be irritated by a roughened surface, etc. For example, the anchoring mechanism 500 may be inserted into a thin or broken pedicle on a spinal vertebrae.

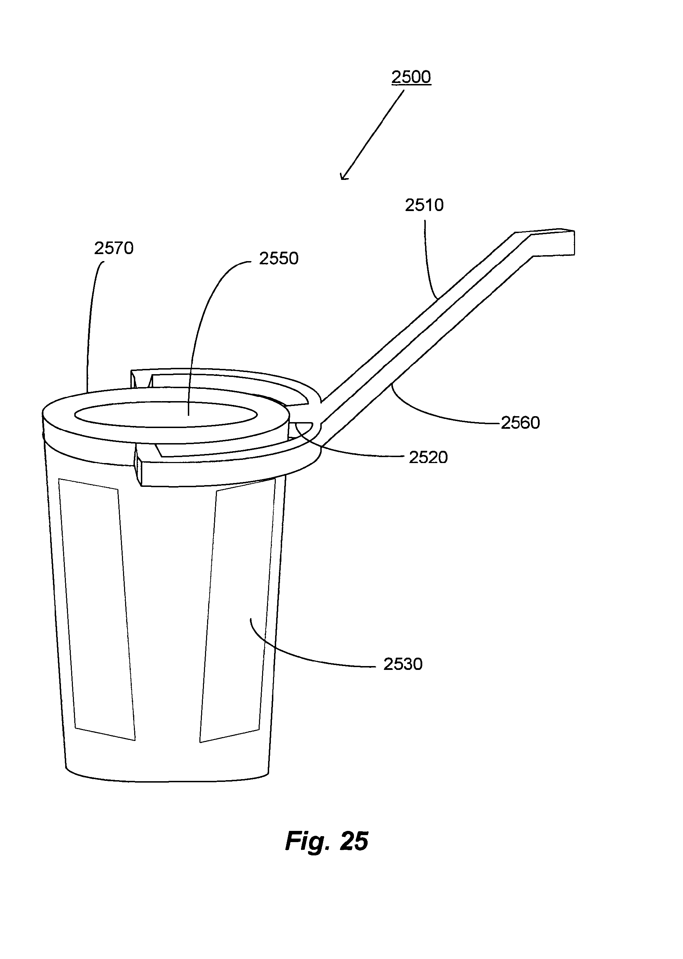

Once the anchoring mechanism 500 is inserted into the pedicle, a screw (bolt, etc.) or pedicle screw may be inserted into the opening 510 of the anchoring mechanism 500. Since turning the screw (bolt, etc.) may apply radial forces, the anchoring mechanism 500 may initially rotate until expanding members push sufficiently of the inside the structural tissue, unless the anchoring mechanism 500 is used in conjunction with some type of anchoring mechanism holder (see Figs. 25-29). Internal threads preformed in opening 510 may also help to minimize the radial forces that may occur when a screw of bolt is inserted into opening 510. The textured or roughened area 530 containing, for example, threads 520 may also limit the initial rotation of the anchoring mechanism but may be subsequently aligned in a direction so as to not irritate or injure an area of the body that may be better served by the smooth surfaced area 540 of the anchoring mechanism 500, for example, the spinal cord, nerve roots, or surrounding tissue where a pedicle cortex becomes breached.

Using the human spine and a spinal cord vertebrae as an example, the smooth portion or area 540 may not irritate the spinal cord, nerve roots, or surrounding tissue as much as the textured or roughened surface area 530. The presence of the smooth portion 540 on the outer surface 515 of the anchoring mechanism 500 facilitates the anchoring mechanism 500 being rotated such that the smooth portion or area 540 is facing the spinal cord, nerve roots, or surrounding tissue rather than the textured or roughened surface area 530. The smooth portion 540 may also be covered with a protective coating composed of, for example, hydroxyl apatite, bone morphogenetic protein, etc., in order to prevent irritation or injury to the spinal cord, nerve roots, or surrounding tissue.

Referring now to Fig. 6, a perspective view of another exemplary embodiment of the anchoring mechanism 600 is provided. The outer surface 610 of the cylindrical body of the anchoring mechanism 600 may be covered with ribs, e.g., 620, which may provide improved grip against the structural tissue where the anchoring mechanism 600 is inserted. The anchoring mechanism 600 may be inserted into, for example, structural tissue before or after a screw (bolt, etc.) is inserted into the opening 630 of the anchoring mechanism 600. Once again, although not show in Fig. 6 (or Figs. 5 and 7) for simplicity, one skilled in the art will understand that the anchoring mechanism may also include one or more expanding members for further increasing gripping or purchase to structural tissue.

Referring now to Fig. 7, a perspective view is shown of still yet another exemplary embodiment of the anchoring mechanism 700. The outer surface 710 of the cylindrical body of the anchoring mechanism 700 may be covered with bumps or studs, e.g., 720, which may provide improved grip against the structural tissue where the anchoring mechanism 700 is inserted. The anchoring mechanism 700 may be inserted into, for example, structural tissue before or after a screw (bolt, etc.) is inserted into the opening 730 of the anchoring mechanism 700. Once again, although not show in Fig. 7 (or Figs. 5 and 6) for simplicity, one skilled in the art will understand that the anchoring mechanism may also include one or more expanding members for further increasing gripping or purchase to structural tissue.

As can be seen from the above description, the anchoring mechanism may include various textured or roughened surfaces using a variety of geometries, e.g., threads, ribs, bumps, studs, etc., that may be over all or a portion of the outer surface of the anchoring mechanism to improve gripping or purchase to the structural tissue into which the anchoring mechanism is inserted. Further, the textured or roughened surface may be applied to the outer surface of expanding members, and in various embodiments may be applied to only the expanding members. In any case, the combination of the textured or roughened surface and the expanding member(s) may result in an instrumentation mounting or screw purchase or gripping that is significantly greater than what might be achieved with a bone or cortical screw alone. This is particularly true in situations where a previously inserted bone or cortical screw has become loose due to wear in the structural tissue channel (e.g., where a patient has osteoporosis or previously had spinal cord vertebras bound or fused together using cortical screws.

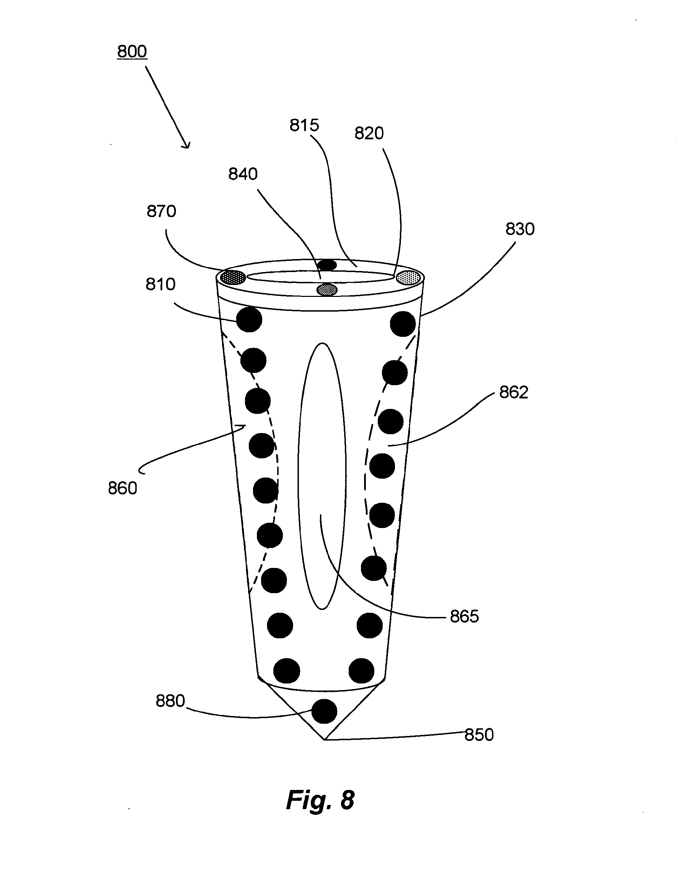

Referring now to FIG. 8, a perspective view of another exemplary embodiment of the anchoring mechanism 800 is provided. The anchoring mechanism 800 may include one or more position and/orientation indicators or markers, e.g., 810, 870, 880, etc. The markers may be made of a material or shape that enables quick identification with electric or sound waves. The anchoring mechanism 800 is shown to include a plurality of expanding members, 860, 862, 865 (fourth one not shown) in an unexpanded form. It is noteworthy that expanding members, 860, 862, 865, etc., have an oval shape. As should be understood, the expanding members, 860, 862, 865, etc. may have a variety of shapes that may work well with the geometry of the anchoring mechanism 800, in this case a cone-like shape (versus a cylinder, etc.). In any case, it may be difficult in certain spinal procedures to determine whether an anchoring mechanism 800 has been positioned properly in the structural tissue, for example in a channel or hole formed in a pedicle of a spinal column bone (e.g., vertebrae). As shown, this embodiment of the anchoring mechanism 800 contains markers 810, 870, 880, etc. to aid in the visualization of orienting the anchoring mechanism 800 properly within structural tissue (e.g., a spinal pedicle). The markers 810, 880, may also be placed on the outer surface 830 of the anchoring mechanism 800, on the inner surface 820 within the interior of the anchoring mechanism 800, or anywhere within the walls of the anchoring mechanism 800, and as such in this case would be particularly benefited by use of x-ray opaque material. Further, to improve lateral length alignment of the anchoring mechanism, the markers, e.g., 810, may be aligned along the length of the anchoring mechanism 800 such that on an x-ray, the location of the opening 840 and end 850 of the anchoring mechanism 800 can be visualized. Positioning of markers on opposite ends, 815 and 850, of the anchoring mechanism 800 may be sufficient to determine and set the lateral length alignment. Furthermore, positioning of markers 810 along the sides of the anchoring mechanism 800 such that the width of the anchoring mechanism 800 can be ascertained on an x-ray may prove beneficial. The markers 810 may also be placed on the expanding wall members 860 of the anchoring mechanism 800 such that the width of the anchoring mechanism 800 with the expanding members expanded may be more accurately seen using, for example, an x-ray or sound waves, so as to ensure that the anchoring mechanism is properly place and expanded to interface sufficiently with the structural tissue. Utilization of the markers 810 may allow the position, orientation, and/or width of the anchoring mechanism 800 to be ascertained once inserted into the structural tissue, e.g., a pedicle, which could allow verification of proper placement inside the pedicle in order to avoid incorrectly placing the anchoring mechanism 800 and screw (bolt, etc.) inside the pedicle or other structural tissue.

The use of visible markers 870 on the rim 815 of the anchoring mechanism 800 in this embodiment may be used to determine the rotational orientation of the anchoring mechanism 800 once inserted into, for example, a structural body. The markers 870 may be different colors, indents, textures, etc. The markers on the upper ring 815 may be made of various colors or shapes (e.g., a letter such as "T" designating, for example, a textured side of the anchoring mechanism) that may be, but need not be, made of an x-ray opaque material to cause the markers to appear on an x-ray. A single marker or multiple markers may be used. Upon insertion of the anchoring mechanism 800 into the pedicle, the markers 870 could be used to determine proper rotational orientation of the anchoring mechanism 800, particularly in the case that the anchoring mechanism 800 is not uniform around its circumference. One example exemplary embodiment was depicted in Fig. 5 where only a portion of the outer surface of the anchoring mechanism 800 is textured, has threads, etc. In such an embodiment, the rotational orientation of the anchoring mechanism 800 would be pertinent in determining whether the smooth portion or the textured or roughened portion of the anchoring mechanism is facing the desired direction or position (e.g., toward or away from a spinal canal).

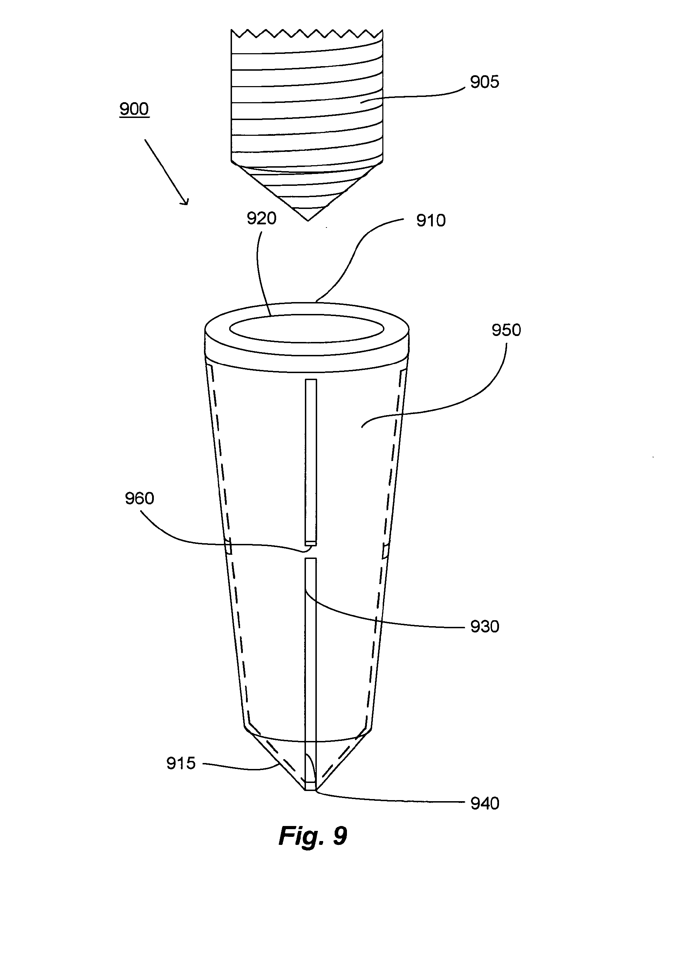

Referring now to Fig. 9, a perspective view of at least one exemplary embodiment of the anchoring mechanism 900 is shown. The anchoring mechanism 900 may be formed in a cone-like shape having a wider opened end 910 and an opposing tip or pointed end 915. The pointed end 915 may include a split tip 940 that may be a continuation of split 930 that extends up for at least a portion of the lateral length 950 of the anchoring mechanism 900. The tip of the anchoring mechanism 900 may be split once or multiple times. The sidewalls of the anchoring mechanism 900 may be attached together with one or more wall connectors 960. As such, these connecting members 960 may be used in order to hold the anchoring mechanism 900 together and prevent premature splitting and/or buckling of the anchoring mechanism 900 during its insertion into structural tissue. The tip 915 may also include one or more connecting members (e.g., a solid portion at the very tip (not shown)) to keep the tip 915 of the anchoring mechanism together, particularly when it is being inserted into structural tissue. Upon insertion of the anchoring mechanism 900 into structural tissue and insertion of a screw (bolt, etc.) 905 into the opening 920 of the anchoring mechanism 900, at least the split 940 in the tip 915 would open more widely and cause the anchoring mechanism 900 to expand, which would cause lateral forces to be increased against the surrounding structural tissue, providing better gripping or purchase of the screw to the structural tissue. Of course, insertion of a screw of sufficient diameter may result in connecting members 960 breaking and the anchoring mechanism walls expanding to cause lateral forces on surrounding structural tissue for improved gripping or purchase.

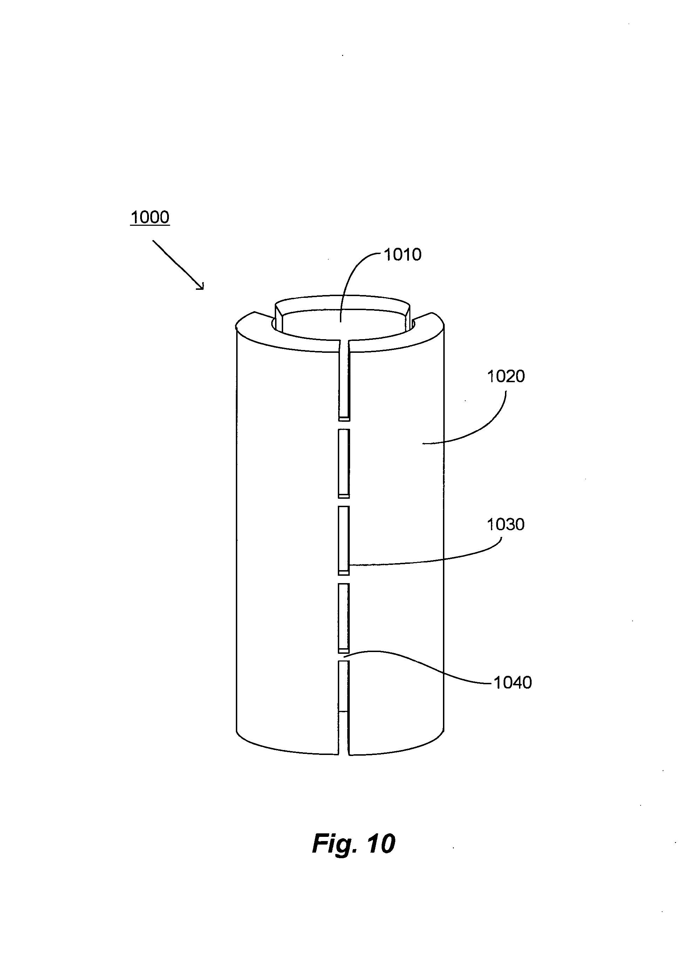

Referring now to Fig. 10, a perspective view of at least one exemplary embodiment of the anchoring mechanism 1000 is shown. The anchoring mechanism 1000 in this embodiment is in the shape of a cylinder and may include one or more split(s) 1030 (in this embodiment shown with three splits) in its side wall(s) 1020 that may be held together with, for example, small connecting members 1040. The anchoring mechanism may be made of a number of different materials that may be malleable or rigid, for example, a rubber, plastic, or metal material including composites, and may be compatible with living tissue(s). The split(s) 1030 may extend across a portion, or the entire lateral length (as shown), of the anchoring mechanism 1000. The anchoring mechanism 1000 may be inserted into, for example, structural tissue. A screw (bolt, etc., not shown) or pedicle screw may be inserted into the opening 1010 of the anchoring mechanism 1000 causing some or all of the connecting members 1040 to disconnect, thereby allowing the anchoring mechanism 1000 to expand and cause increased lateral forces against the surrounding structural tissue so as to increase gripping, purchase or attachment compared to using only a screw or pedicle screw to attach be inserted into the surrounding structural tissue. As such, the lateral forces of the screw being inserted into opening 1010 may cause the diameter of the anchoring mechanism 1000 to expand outward (which may or may not cause one or more connecting members 1040 to break) thus causing the interface, gripping and/or purchase between the outer surface of side wall 1020 of the anchoring mechanism 1000 and the surrounding structural tissue to increase. In various embodiments a screw geometry (rod, tapered, etc.) and size may be selected to best fit the final expanded shape of the anchoring mechanism 1000 to a predetermined hole or channel shape in the structural tissue. Further, the combination of the size of the anchoring mechanism and the size and shape (rod, tapered, etc.) of the screw or pedicle screw may be selected so that only some of the connecting members 1040 break when the side walls 1020 are expanded, so that at least on connecting members 1040 between each of the adjacent walls remain connected. The screw and anchoring mechanism 1000 may be pre-threaded or self tapping.

Referring now to Fig. 11, a top view 1000 of a spinal vertebra 1105 incorporating one exemplary anchoring mechanism 1150, according to at least one embodiment of the present invention is provided. The anchoring mechanism 1150 may be inserted into a channel 1145 in structural tissue, for example, one pedicle 1115 of the vertebra 1105. A screw (bolt, etc.) 1140 may be introduced into the top opening of the anchoring mechanism 1150 before or after insertion of the anchoring mechanism 1150 into a channel 1145 in structural tissue, then subsequently screwed or forced almost fully into the anchoring mechanism 1150 so that the head of the screw or bolt 1140 extends from the anchoring mechanism 1150 at a desired length, for example, sufficient to attach various instrumentality for vertebra fixation. According to at least one embodiment of the present invention, the anchoring mechanism 1150 may be constructed such that a wall member(s) may expand laterally upon partial or full screw (bolt, etc.) 1140 insertion. Such expansion may result in increased purchase or grip between the anchoring mechanism 1150 and the structural tissue, for example, the pedicle 1115 of vertebra 1105. In this manner, screw purchase may be augmented by the lateral forces applied by expansion of at least a portion of the anchoring mechanism 1150 upon screw (bolt, etc.) 1140 insertion. As one skilled in the art would understand, various lengths, diameters, and geometries of anchoring mechanism 1150 may be used in the present embodiment, even though a particular one is shown in this figure.

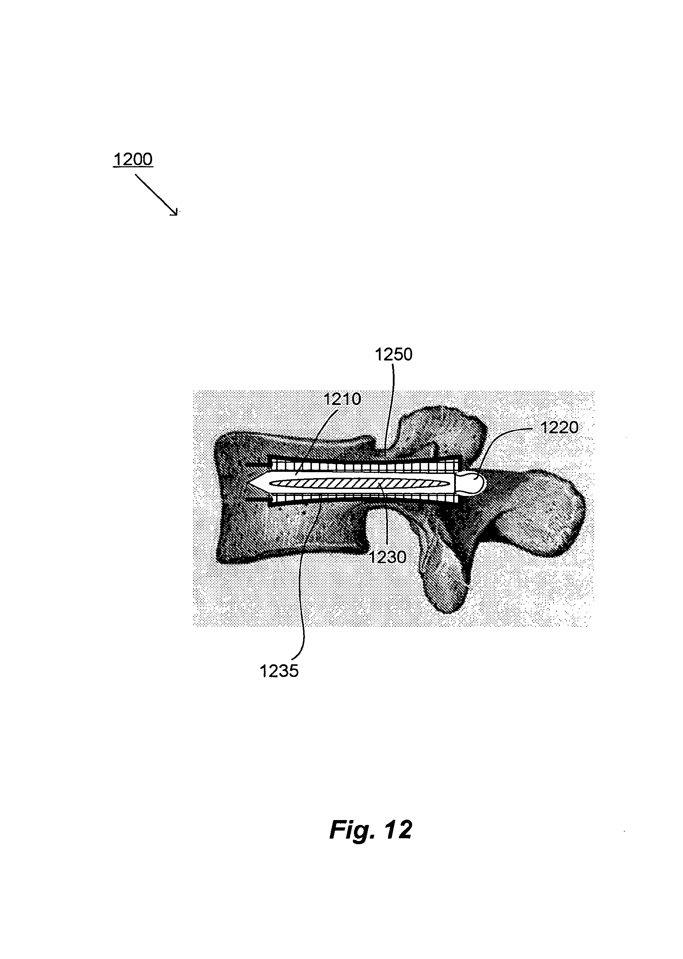

Referring now to Fig. 12, a side view of a vertebra 1200 incorporating one exemplary embodiment of the anchoring mechanism 1210 according to the present invention is provided. The anchoring mechanism 1210 may be inserted into structural tissue, for example, into and through a pedicle 1250 of a vertebra 1200 and reaching into the vertebral body. A screw (bolt, etc.) 1220 may be introduced into one end (e.g., an open end) of the anchoring mechanism 1210. According to at least one embodiment of the present invention, the anchoring mechanism 1210 may be constructed such that one or more wall members, e.g. expandable wall member 1230, may expand laterally upon screw (bolt, etc.) 1220 insertion. There may be, for example, any number of expandable wall members such as three, four, or five, expandable wall members that may be equally spaced around the diameter of the anchoring mechanism 1210. As shown, the expandable members may expand differing amounts such that there is less expansion in the pedicle and more expansion in the vertebral body. Such expansion may result in increased purchase or grip between the anchoring mechanism 1210 and structural tissue, for example, between the pedicle 1250 of a vertebra 1200 and the screw 1220. This is particularly beneficial when a pedicle screw 1220 previous inserted is too loose or the channel 1235 in the pedicle 1250 is worn to be to large to properly attaché the screw 120 thereto. In this manner, screw purchase may be augmented by the lateral forces applied by expansion of wall members 1230 of the anchoring mechanism 1210 upon partial to full screw (bolt, etc.) 1220 insertion into the anchoring mechanism 1210.

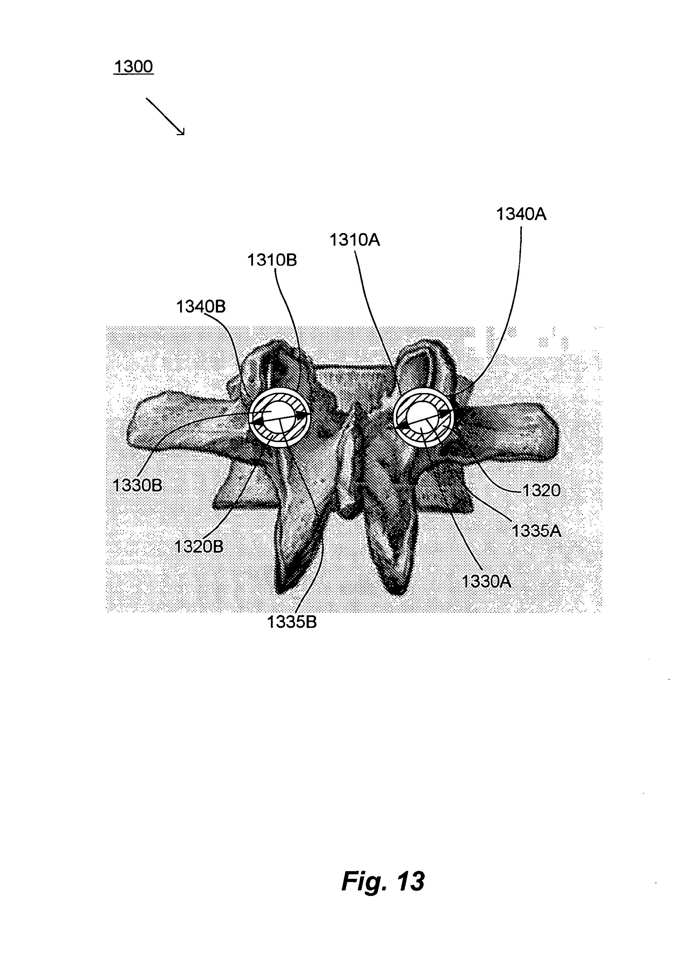

Referring now to Fig. 13, a posterior view of a vertebra 1300 incorporating one exemplary embodiment of the anchoring mechanism 1320 is provided. Anchoring mechanisms 1320A and 1320B may be inserted into a pedicles 1340A and 1340B of a vertebra, having formed therein pedicle channels 1310A and 1310B. Screws (bolts, etc.) (not shown) may be introduced into the openings 1330A and 1330B of anchoring mechanisms 1320A and 1320B, respectively, to thereby expand at least a portion of the outer diameters 1335A and 1335B of the anchoring mechanism 1320A and 1320B to thereby increase gripping or purchase with the pedicles.

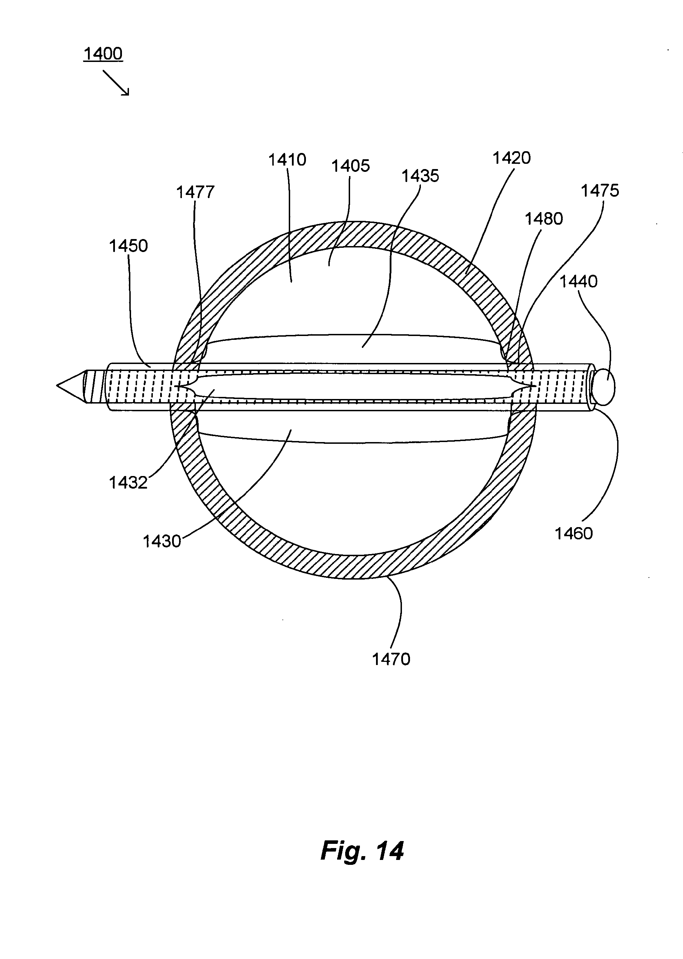

Referring now to Fig. 14, a cross-sectional view of an exemplary long bone embodiment 1400 incorporating one exemplary anchoring mechanism 1450 is provided. According to at least one embodiment of the present invention, the anchoring mechanism 1450 may be constructed such that a wall member(s) 1430, 1432, 1435, etc. may expand laterally upon insertion of a screw (bolt, etc.) 1440 into an opening 1460 located on one end of the anchoring mechanism 1450. First, an anchor mechanism 1450 having expandable members 1430, 1432, 1435, etc., may be inserted through two holes 1475 and 1477 and a channel formed through an area of a long bone 1470 (cross-section of the long bone 1405 shown hear cut along the middle of holes 1475 and 1477). A screw (bolt, etc.) 1440 may be at least initially partially introduced into the anchoring mechanism 1450 and then screwed or forced into the anchoring mechanism 1470 to force outward the expanding wall members 1430, 1432, 1435, etc. The expanding wall members 1430, 1432, 1435, etc., may have a geometry such that only certain smaller portions, e.g., at location 1480 of the anchoring mechanism, are squeezed in the interface between the anchoring mechanism 1450 and the holes 1475 and 1477 made in the long bone 1405. This would provide a very tight and almost certain secure fit of the anchor mechanism within the sidewalls of the long bone 1420. In one variation, the anchoring mechanism 1450 may include markers on the surface near opening 1460 that may help determine radial positioning and there may be only one or two expanding members 1430 and/or 1435. This may provide sufficient gripping or purchase, particularly in a long bone 1405, given the circular through holes 1475 and 1477 in the cortical bone portion 1420 being formed to only provide enough space to insert there through the anchoring mechanism 1450 before expansion. In another variation, the length of the expanding member 1430, 1432, 1435, etc., may be made shorter in order to prevent significant pressure and/or damage to the long bone 1405 when the wall members' 1430 are in an expanded position. Such designs may be particularly beneficial to fragile of thin walled bones, for example, bones of people with osteoporosis. Regardless, such expansion of expandable members 1430, 1432, 1435, etc., may result in increased purchase, grip, or interdigitation between the anchoring mechanism 1450 and, for example, the cancellous bone 1410 of a long bone 1405. In this manner, screw purchase may be augmented by the lateral forces applied by expansion of the wall members 1430 of the anchoring mechanism 1450 upon screw (bolt, etc.) insertion. According to at least one embodiment of the present invention, the anchoring mechanism 1450 may increase screw pullout strength, thus reducing the likelihood of screw pullout, by interdigitating with cortical bone portion 1420 in one or more locations along the cortical surface 1470 of, for example, a long bone 1405.

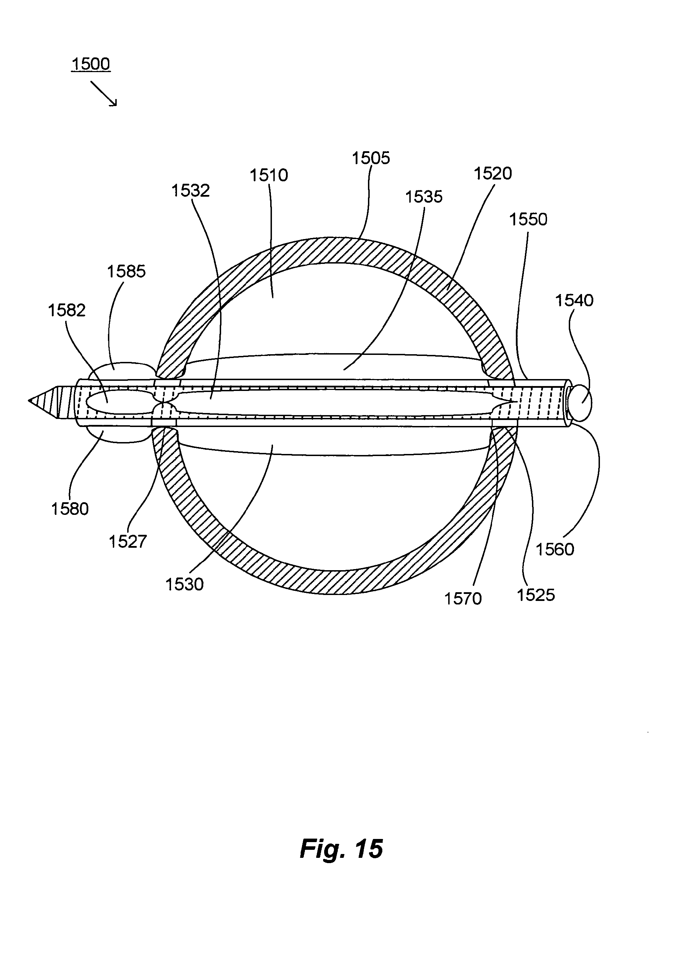

Referring now to Fig. 15, a cross-sectional view 1500 of a long bone 1505 incorporating one exemplary embodiment of the anchoring mechanism 1550 is provided. According to at least one embodiment of the present invention, the anchoring mechanism 1550 may be constructed such that at least a first portion 1530 and a second portion 1580 of at least one expanding member may expand laterally upon insertion of a screw (bolt, etc.) 1540 into an opening 1560 located at one end of the anchoring mechanism 1550. The expanding wall member(s) 1530, may be constructed such that at least one end, e.g., portion 1570 are cut to fit close to the through hole 1525 interface between the anchoring mechanism 1550 and the long bone 1505 in order to reduce stress and possible damage to the long bone 1505 during the wall members' 1530 expansion. The expansion of the first portion 1530 may result in increased purchase, grip, or interdigitation between the anchoring mechanism 1550 and, for example, the cancellous bone 1510 of a long bone 1500. In this manner, similar to the embodiment shown in Fig. 14, screw 1540 purchase may be augmented by the lateral forces applied by expansion of the first portion 1530 of the anchoring mechanism 1550 upon screw (bolt, etc.) 1540 insertion. The expanded second portion(s) 1580, 1582, 1585, etc. may form any geometric shape as long as at least some of the outer side(s) of the expanded second portion(s) 1580, 1582, 1585, etc. are larger than the space between through hole 1527 in the cortical bone 1520 through which the anchoring mechanism 1550 extends. The expansion of the second portion(s) 1580, 1582, 1585, etc., may result in even further increased pullout strength by expanding to a dimension greater than the hole 1527 in the cortical bone 1520 through which the anchoring mechanism 1550 extends.

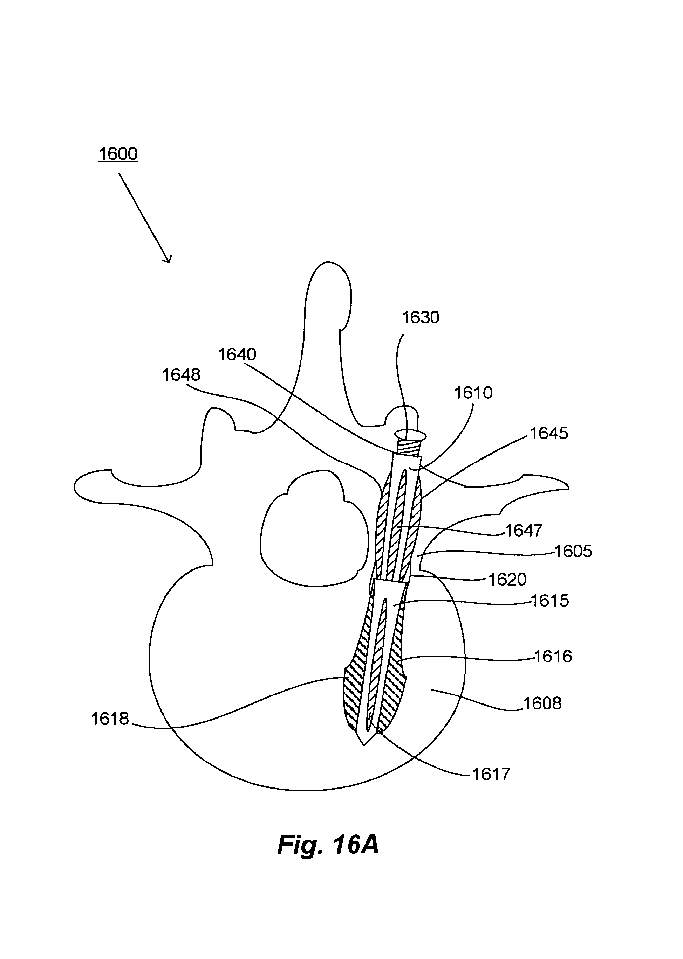

Fig. 16A shows a top view of a vertebra 1600 including one exemplary embodiment of anchoring mechanism 1610 cascaded in series with anchoring mechanism 1615. Over time, the pedicle channel 1620 of a previous pedicle screw (not shown) may become worn such that the previous pedicle screw is no longer positioned firmly. According to at least one embodiment, the anchoring mechanism 1610 may be inserted into the previously used pedicle channel 1620. In this example, the pedicle channel 1620 and vertebral body mounting holes may be worn approximately evenly along its entire length or depth in the pedicle 1605 and vertebral body 1608. A screw (bolt, etc.) 1630 may be inserted fully into an opening 1640 formed in one end of the anchoring mechanism 1610 after the anchoring mechanism 1610 is inserted into the pedicle channel 1620 and anchoring mechanism 1615 is inserted into vertebral body 1608, which may thereby cause the expandable wall members 1616, 1617, 1618, 1645, 1647, 1648, etc. (for example, four expandable wall members formed symmetrically around the circumference of each of the anchoring mechanisms 1610 and 1615, respectively) of the anchoring mechanisms 1610 and 1615 to expand radially or laterally. The screw (bolt, post, etc.) 1630 may reach through the most of or the entire length of both anchoring mechanism 1610 and anchoring mechanism 1615. As shown, the expandable wall members 1616, 1617, 1618, 1645, 1647, 1648 may be expanded at a distance that is relatively uniform and each various equally along the lateral length of the anchoring mechanisms 1610 and 1615 because the wear out of the pedicle channel 1620 is relatively the same throughout it length or depth in the pedicle. Expansion of the wall members 1616, 1617, 1618, 1645, 1647, 1648, etc. may cause increased purchase or grip or interdigitation between the anchoring mechanisms 1610 and 1615 and the walls of the previously used pedicle channel 1620 and vertebral body. It is noteworthy that each of the anchoring mechanisms 1610 and 1615 may have different shaped expandable wall members from each other. In any case, the increased screw purchase may restore the strength of screw 1630 in the pedicle channel 1620 and vertebral body 1608 to its original initial pull-out strength (prior to channel 1620 and vertebral body hole ware) or greater, but at least to a pull-out strength sufficient to function for its intended purpose (e.g., spinal vertebra fixation). Although this embodiment shows the use of two anchoring mechanisms aligned in series, a single longer anchoring mechanism may be used. Further, the two anchoring mechanisms may be held together with an interlocking means or a holding and aligning means, for example, a malleable boot that fits over the bottom end of anchoring mechanism 1610 and the top end of anchoring mechanism 1615 that are butted together so as to ensure proper alignment and to hold anchoring mechanism 1615 in place with anchoring mechanism 1610.

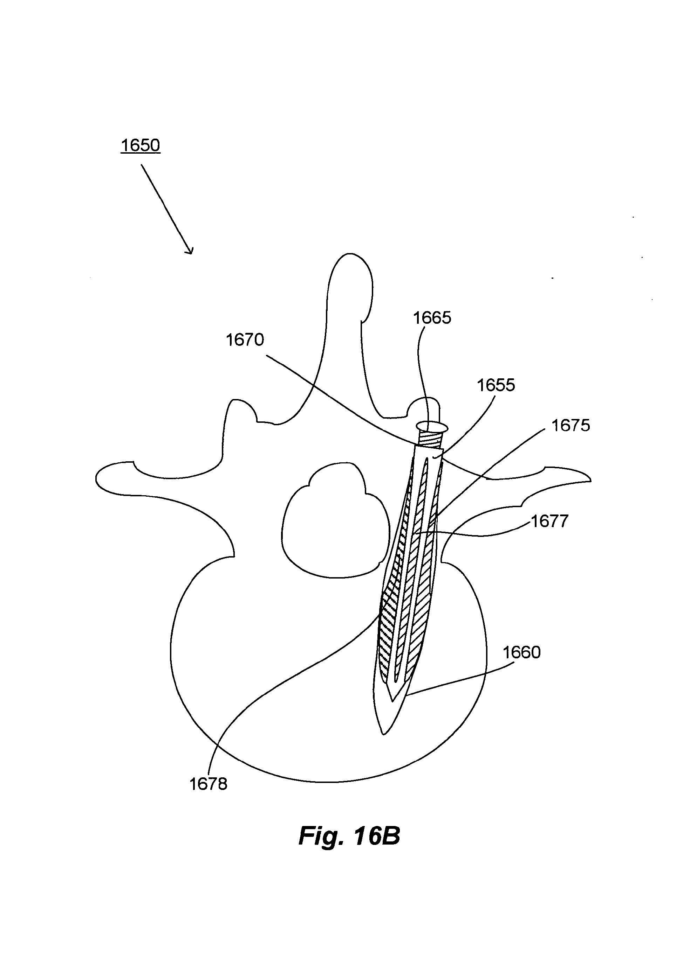

Referring now to Fig. 16B, a top view of a vertebra 1650 including one exemplary embodiment of an anchoring mechanism 1655 is provided. This embodiment depicts, for example, a worn pedicle channel and vertebral body anchoring hole 1660 where the inner deeper portion(s) of the pedicle channel and vertebral body anchoring hole 1660 has become more worn (or accidentally formed wider) than the portion near the opening 1670 over time through, for example, use of spinal vertebra fixation instrumentation that through everyday use experiences more stress internally as a result of, for example, everyday human body movement and the principle of leveraging about a point (outer hard bone in the pedicle). In this example, when the screw (post, bolt, etc.) 1665 is inserted fully into an opening 1670 formed in one end of the anchoring mechanism 1655 after the anchoring mechanism 1655 is inserted into the pedicle channel 1660, which may thereby cause the expandable wall members 1675, 1677, 1678, etc. (for example, four expandable wall members formed symmetrically around the circumference of the anchoring mechanism 1655) of the anchoring mechanism 1655 to expand radially or laterally more at the bottom of the pedicle channel 1660. The anchoring mechanism 1655 may have expanding wall members 1675 that may expand upon insertion of a screw (post, bolt, etc.) 1665 to fit the worn pedicle channel and vertebral body anchoring hole 1660 to increase purchase or grip or interdigitation between the anchoring mechanism 1655 and the walls of the previously used pedicle channel and vertebral body anchoring hole 1660. The increased screw purchase may restore the strength of the pedicle channel and vertebral body anchoring hole 1660 to screw adhesion to its prior strength, or at least such that the function of the previous pedicle screw may be restored (or sufficient strength if the hole is mis-formed initially). Note that in this embodiment a single longer anchoring mechanism 1655 has been shown, which has expandable wall members that are not symmetrical along their length. Of course, multiple anchoring mechanisms could have been used instead.

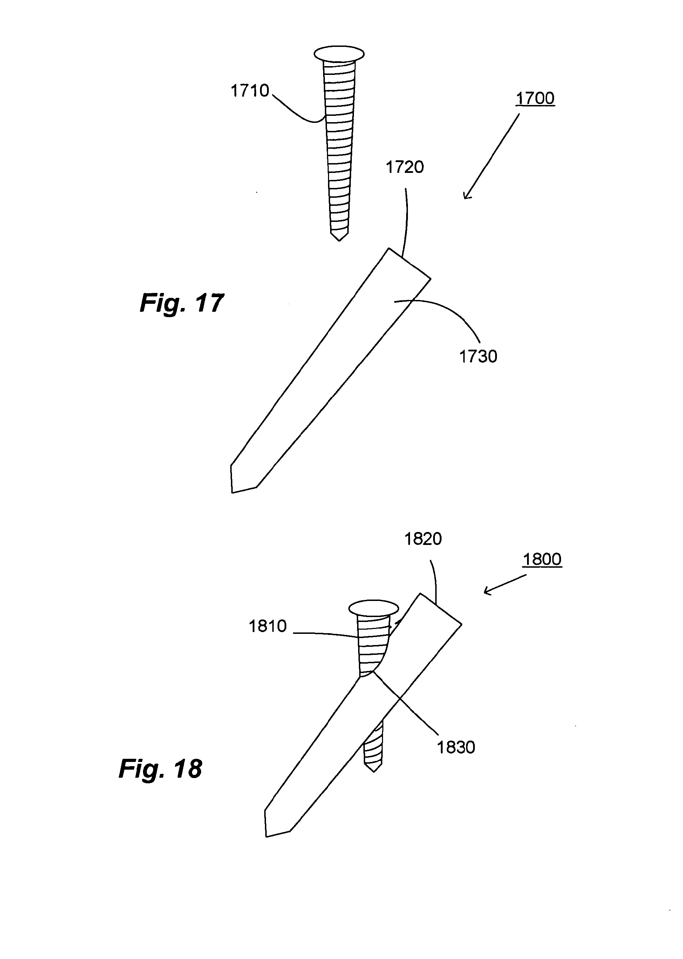

Referring now to Fig. 17, a perspective view 1700 is provided of one exemplary embodiment of the anchoring mechanism 1730 and a screw 1710. The screw (bolt, etc.) 1710 has not been inserted into the anchoring mechanism 1730. In some cases, a pedicle channel may become worn in a manner oblique to the original path of the pedicle channel. An angle, e.g., an oblique angle, insertion point for the screw (bolt, etc.) into the anchoring mechanism may be desirable in order to allow maximum gripping of a well-worn or incorrectly formed hole or pedicle channel. In such cases, the anchoring mechanism 1730 may be placed at an angle relative to the positioning of the screw (bolt, etc.) 1710 so that a desired orientation of the screw 1710 will occur in the bone or pedicle and the anchoring mechanism 1730 may provide more gripping or purchase due to its angled orientation. While typically screws (bolts, etc.) are inserted through the opening 1720 of an anchoring mechanism 1730, this embodiment would allow the insertion of a screw (bolt, etc.) 1710 at an angle point, e.g., an oblique angle, different from normal lateral entry in the opening 1720 of the anchoring mechanism 1730. The anchoring mechanism 1730 may be drilled and/or tapped at a desired point of angular entry in, for example, a side or even the opening 1720, at an angle relative to the lateral axis of the anchoring mechanism 1730, to provide an insertion point. The screw (bolt, etc.) 1710 may then be inserted into the anchoring mechanism 1730 at this entrance point and angle. In various embodiments, the anchoring mechanism 1730 may be made of a malleable material such as plastic or rubber and the screw may be made of a rigid material such as metal, steel, etc., such that the screw (bolt, etc.) 1710 may be inserted without tapping or drilling. The malleable material may also result in expansion of the anchoring mechanism 1730 so as to further increase grip between the screw (bolt, etc.) and the anchoring mechanism 1730.

Referring now to Fig. 18 shows a perspective view of another exemplary embodiment of the anchoring mechanism 1800 having a screw 1810 insert at an angle relative to the lateral axis of the anchoring mechanism 1800. The screw (bolt, etc.) 1810 has been inserted into the anchoring mechanism 1800 at, for example, point 1830 so as to be at an angle (e.g., an oblique angle) relative to putting the screw 1810 into the opening 1820 at an lengthwise orientation consistent with the lateral axis of the anchoring mechanism 1800. This embodiment may be useful in the case that a previous pedicle channel has become significantly worn through constant abuse caused by everyday human body activity or through poor initial forming of the pedicle channel, such that the desired or intended angle of the pedicle channel has not been maintained or achieved. The anchoring mechanism 1800 may be placed such that it matches the angle of the worn or misguided pedicle channel, and the screw (bolt, etc.) 1810 may be placed at the proper angle as intended by the original pedicle channel to enable proper use, for example, with vertebra fixation instrumentation or hardware. The anchoring mechanism 1800 may be tapped or drilled at a point 1830 at an angel (e.g., an oblique angle) even at the opening 1820 of the anchoring mechanism 1800. The anchoring mechanism 1800 may be made of a malleable material such that the screw (bolt, etc.) 1810 may be inserted by self-tapping without prior tapping or drilling. In such a configuration, the malleable material may further increase grip between the screw (bolt, etc.) and the anchoring mechanism 1800 as well as providing some expansion characteristics to better grip the structural material. Further, the angle orientation made between the anchoring mechanism 1820 and screw 1810 may create a wide wedging type of configuration (e.g., an X or T shape) not obtainable by simply placing the screw 1810 straight into main lateral axis of the anchoring mechanism 1820, so as to significantly increase purchase and reduce or eliminate any further pedicle channel wear.

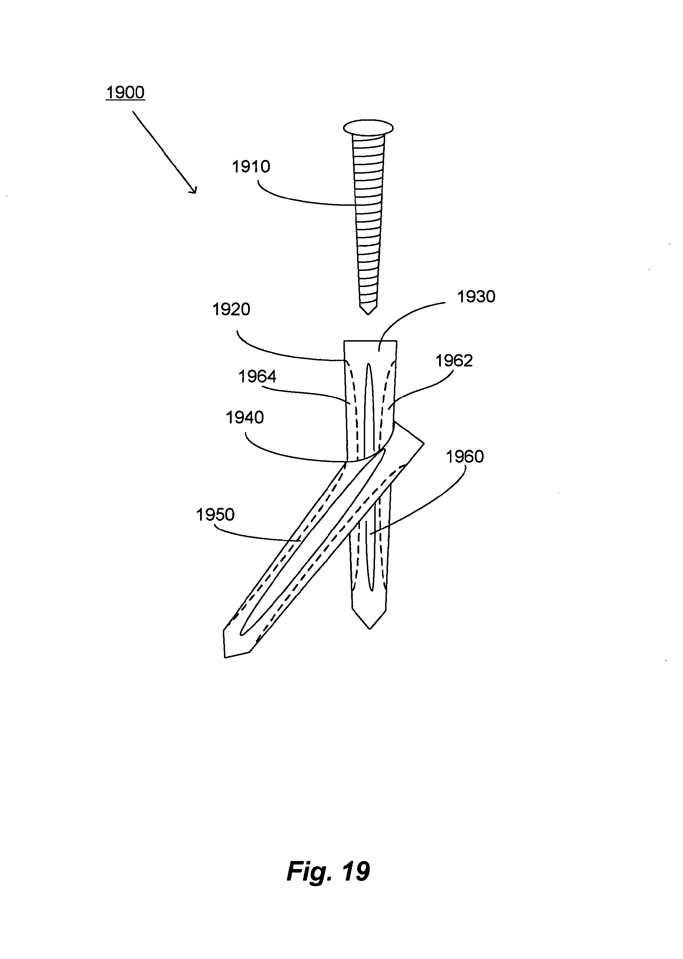

Referring now to Fig. 19, a perspective view 1900 of one exemplary embodiment using multiple anchoring mechanisms is shown. A first anchoring mechanism 1950 is provided with a complementary second anchoring mechanism 1930. In this case, a first anchoring mechanism 1950 allows for the insertion of a screw (bolt, etc.) 1910 into the second anchoring mechanism 1930. The first anchoring mechanism 1950 may be made of a malleable material in order to more easily allow the second anchoring mechanism 1930 to be inserted directly or obliquely into the first anchoring mechanism 1950, and provide some expandable characteristic that may help when the screw (bolt, etc.) 1910 is inserted into the second anchoring mechanism 1930. First, the first anchoring mechanism 1950 may be inserted into a worn pedicle channel (with or without a hole 1940 made therein). This embodiment may be useful because a pedicle channel may become worn over time by, for example, constant stress and movement that may occur due to everyday human body activity when spinal fixation instrumentation is used to hold or fuse together adjacent vertebra. To the extent that the original angle of the pedicle channel has not been maintained or properly formed and is relatively severe so that a single anchoring mechanism may not be sufficient. Insertion of the second anchoring mechanism 1930 into the first anchoring mechanism 1950 may allow a physician to correct the improperly positioned angle of, for example, a pedicle channel that may have resulted from a worn pedicle channel. This correction could be accomplished by using the first anchoring mechanism 1950 to fill the region where the pedicle channel has become most seriously worn and act as a more stable or fill structure for the insertion of the second anchoring mechanism 1930, which may be used for proper positioning and orientation of the pedicle screw 1910. The second and subsequent anchoring mechanism 1930 may be inserted, for example, at a any point along the lateral length of the first anchoring mechanism 1930, such as point 1940. A screw (bolt, etc.) 1910 or pedicle screw may then be inserted into the opening 1920 of the second and subsequent anchoring mechanism 1930. The walls, for example expandable walls 1960, 1962, 1964, etc., of the second anchoring mechanism 1930 may expand upon insertion of the screw (bolt, etc.) 1910 or pedicle screw causing increased screw gripping, purchase and/or attachment between the subsequent anchoring mechanism 1930, the first anchoring mechanism 1950, and the structural tissue. Further, the angle between the first anchoring mechanism 1950 and second anchoring mechanism 1930 may create a wide wedging type of configuration (e.g., an X or T shape) not obtainable with a single anchoring mechanism so as to significantly increase purchase and reduce or eliminate any further pedicle channel wear.

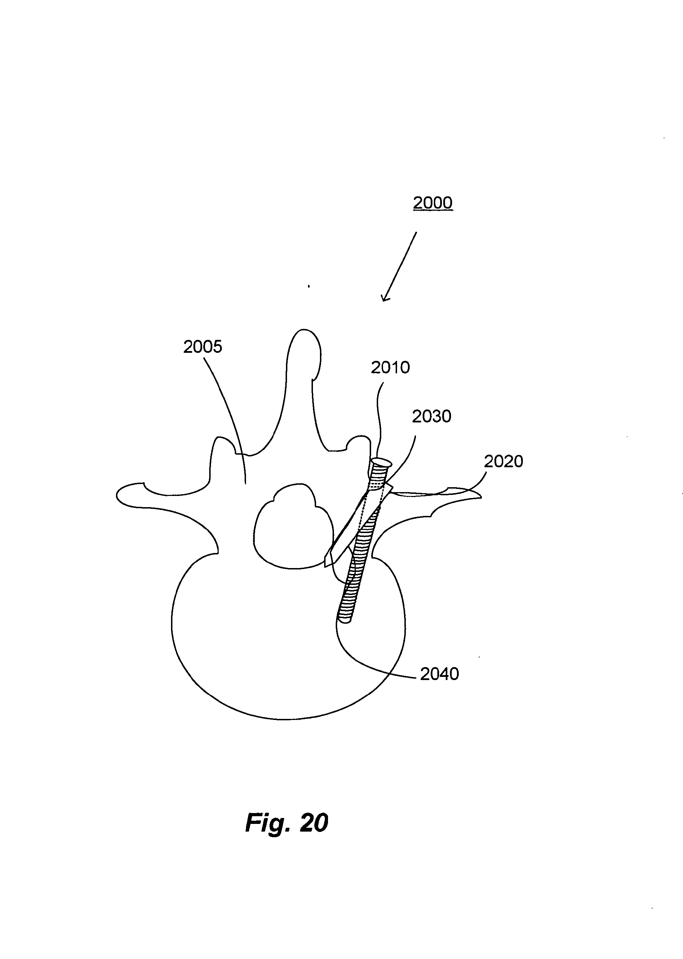

Referring now to Fig. 20, a top down view 2000 of a vertebra 2005 with an exemplary anchoring mechanism 2020 and angled screw 2010 inserted into a vertebra 2005 are provided. The pedicle channel and/or vertebral body anchoring hole 2040 may be worn such that the intended original angle of the pedicle channel and/or vertebral body anchoring hole 2040 has not been maintained and placing the anchoring mechanism 2020 therein with a screw 2010 in axial alignment with its lateral axis will result in an improperly positioned or insecure pedicle screw or bone screw. Thus, the anchoring mechanism 2020 may be inserted such that it follows the resulting angle and/or path of a worn pedicle channel and/or vertebral body anchoring hole 2040 (or improperly formed channel) of a previously inserted pedicle screw, which may increase screw purchase between the anchoring mechanism 2020 and the worn pedicle channel and/or vertebral body anchoring hole 2040. The screw (bolt, etc.) 2010 may be inserted into the anchoring mechanism 2020 at an angle that may be oblique to the opening 2030 of the anchoring mechanism 2020, which may thereby allow the screw (bolt, etc.) 2010 to be placed at a proper angle for use with, for example, spinal plates or other instrumentation while achieving improve gripping, purchase or anchoring of the screw 2010 in the structural tissue (e.g., pedicle area and vertebral body of vertebra 2005).

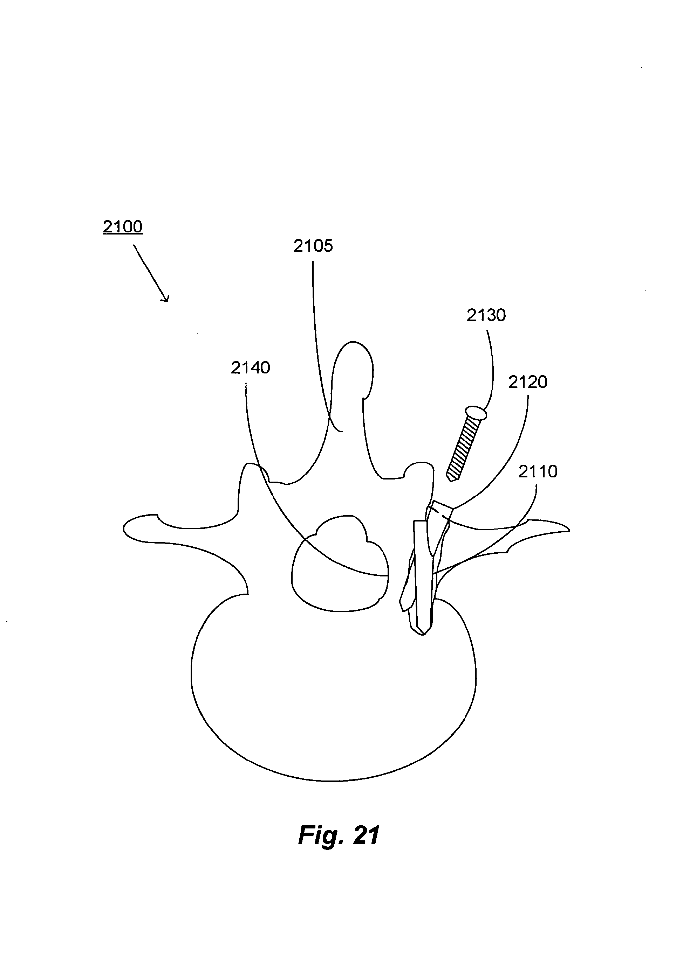

Referring now to Fig. 21, a top down view 2100 of a vertebra 2105 including an exemplary embodiment of a first anchoring mechanism 2110 inserted into a pedicle 2140 and a second anchoring mechanism 2120 placed into at least a portion of the first anchoring mechanism to repair or reconstruct a situation where the pedicle channel is incorrectly positioned and/or angled. The original angle of the pedicle channel may no longer be maintained, which may lead to an insecurely or improperly placed pedicle or bone screw if only a single anchoring mechanism were used. Rather, the first anchoring mechanism 2110 may be first inserted into the worn or improperly formed pedicle channel such that purchase between the first anchoring mechanism 2110 and the surrounding structural tissue may be increased or a large open area void of structural tissue may be filled. The subsequent anchoring mechanism 2120 may then be inserted into the first anchoring mechanism 2110 at an angle, for example, an oblique angle, relative to the lateral axis of the first anchoring mechanism 2110. This angular insertion of the subsequent anchoring mechanism 2120 may allow increased purchase between the anchoring mechanisms, e.g., 2110, 2120, and the surrounding structural tissue so that a desired screw orientation and increased purchase may be maintained or achieved. In this way, restoring the proper angle by creating a desired insertion point for the second anchoring mechanism 2120 may achieve correct screw (bolt, etc.) 2130 angle into the pedicle channel. Variations of this configuration may be used to redirect screw orientation and obtain required gripping and purchase so as to achieve, for example, screw (bolt, etc.) 2130 entry the pedicle 2140 at the proper angle. Additional subsequent anchoring mechanisms 2120 (more than two) may be added before insertion of the screw (bolt, etc.) 2130 or pedicle screw as one skilled in the art may determine are needed.

Referring now to Figs. 22A, 22B, and 22C, variations in the shapes of various pedicle channels may be seen. Referring now to Fig. 22A, a top down view of a vertebra 2200 is shown with a worn or improperly formed pedicle channel 2210. The pedicle channel 2210 has been worn or shaped such that the opening of the pedicle channel 2220 is larger or more worn than the interior or lower depth 2230 of the pedicle channel 2210. FIG. 22A depicts what may be one common way in which pedicle channels 2210 may become worn or formed initially.

Referring now to Fig. 22B, a top down view of a vertebra 2250 is shown with a worn or improperly formed pedicle channel 2255. The pedicle channel 2255 has been formed or worn such that the interior or deepest part of the pedicle channel 2265 is larger, wider, or more worn than the opening of the pedicle channel 2260. Fig. 22B depicts what may be another common way in which pedicle channels 2255 become worn or formed initially.

Referring now to Fig. 22C, a top down view of a vertebra 2275 is shown with a worn pedicle or improperly formed channel 2280. The pedicle channel 2280 has been formed or worn such that the interior of the pedicle channel 2265 and the opening of the pedicle channel 2285 have both been formed or worn to be larger than, for example, a screw diameter. Fig. 22C depicts what may be another common way in which pedicle channels 2280 become improperly formed initially. The various embodiments of anchoring mechanisms disclosed herein help address the various worn or improperly formed pedicle channels shown in Figs. 22A - 22C, so as to increase gripping, purchase, or attachment of a screw to structural tissue.

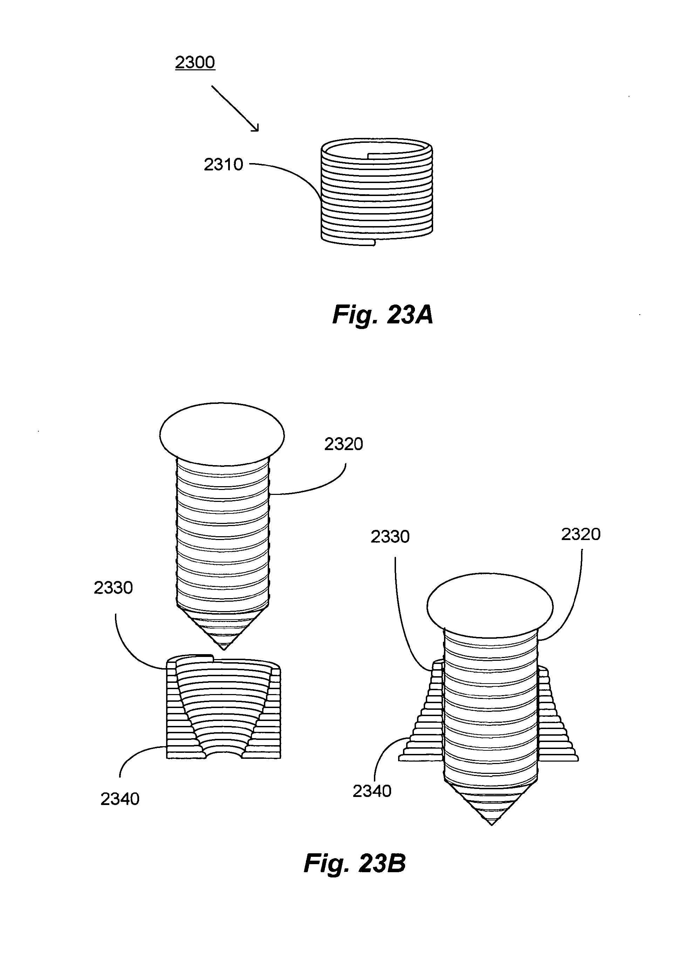

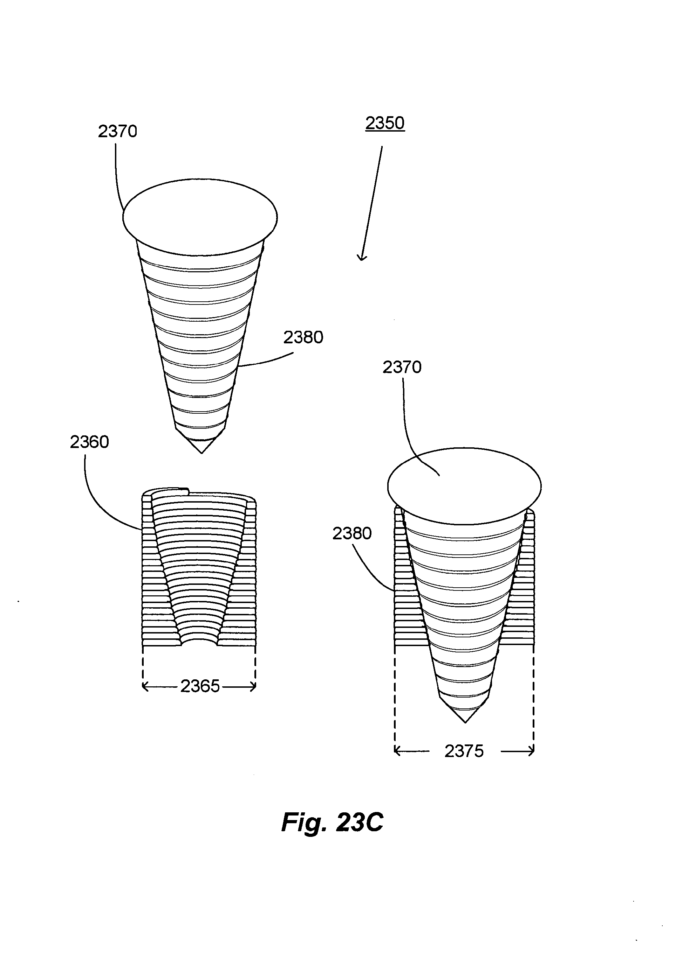

Referring now to Figs. 23A, 23B and 23C, various coil shaped anchor mechanism are shown. Referring to Fig. 23A, a perspective view of one exemplary embodiment of a coil shaped anchor mechanism 2310, e.g., a helical coil, for increasing screw grip, purchase, or attachment is shown. The coil 2310 may be made of a spring type material such as spring steel or may be made of a more malleable material (having spring characteristics) to reduce irritation to surrounding structural tissue. A malleable material may also be used for coil 2310 to allow a screw (bolt, etc.) to be more easily inserted directly therein and may increase purchase or gripping between the screw (bolt, etc.) and the helical coil 2310. The coil 2310 may be constructed so as to include a biocompatible coating to improve acceptance by living cells of the structural tissue. In the event that the coil 2310 may be used in a vertebra pedicle, and there is evidence that a pedicle wall has been breached, the coil 2310 may be constructed of a malleable material and/or contain a biocompatible coating that will not irritate or harm the surrounding nerve tissue adjacent to the point where the pedicle wall has been breached. However, care will need to be used to make sure that the coil 2310 does not start uncoiling and pinch a nerve fiber between itself and the pedicle. This risk may be reduced by including markers on the coil anchor mechanism 2310 or making it from an x-ray wave or sound wave reflecting material.

Referring now to Fig. 23B, a cross-sectional view of one exemplary coil 2330 shape and perspective view of a screw (bolt, etc.) 2320 to be inserted into the helical coil 2330 in at least one exemplary embodiment. The coil 2310 may be inserted into a pedicle channel to increase screw grip, purchase, or attachment with the surrounding structural tissue. In this exemplary embodiment, the various coils 2340 of the coil 2310 may become progressively thicker along the lateral length of the coil 2310 from an opening where the screw 2320 may be first inserted to an end where the screw 2320 may finally exit. Further, the screw 2320 may have a cylindrical shape with parallel walls and a pointed end. This combination of progressive thickness of the coil 2330 and straight parallel walls of the screw 2320 may cause the coil 2310 to expand upon insertion of a screw (bolt, etc.) 2320, which would increase screw grip, purchase, or attachment of the screw 2320. As seen, once the screw 2320 having a diameter approximately the same size as the opening in the top of the coil 2330 is inserted, by for example turning the screw 2320 so that is screws into the coil 2330, the coil will remain approximately the same width or diameter on top but will swell or expand considerably at the bottom end and result in an angled outer diameter from smaller to larger. This shape may be particularly suitable for the pedicle channel 2255 shape shown in Fig. 22B. The coil 2310 may be a helical coil and may be constructed of a biocompatible material(s), for example, allograft, polyetheretherkeetones (PEEK), carbon fiber, etc. Again, the coil 2310 may be constructed of a metal and/or malleable material. The various coils 2340 of the helical coil may be made to include or coated with hydroxyl apatite, bone morphogenetic protein, etc.

Referring now to Fig. 23C, a perspective cross-sectional view of another coil 2360 and screw (bolt, etc.) 2370 combination is shown. In this example, a tapered side or cone shaped screw 2370 may be inserted into the coil 2360 (e.g., a varying wall thickness helical coil). The sides of the screw (bolt, etc.) 2380 may be tapered and large enough in diameter so that insertion of the screw (bolt, etc.) 2370 causes the coil 2360 to expand evenly. The sides of the screw (bolt, etc.) 2380 may be tapered such that the insertion of the screw (bolt, etc.) 2370 may cause a less tapered expansion, or even a straight outer wall, of the expanded coil 2360 so that this coil 2360 and screw combination may be more appropriately used in a relatively evenly but wide hole in structural tissue or channel in a pedicle of a vertebra as shown in Fig. 22C. As shown, when the coil 2360 is used with a screw 2370 having sufficiently large diameter the entire unexpanded width 2365 of the coil 2360 may be increase evenly along its entire lateral length to a larger diameter 2375 (as shown with expanded coil 2380). Some exemplary diameters may be, for example, 3mm to 10 mm, having a typical range of 4 mm to 8 mm in diameter. Some exemplary lengths of screws may be in the range from 25 mm to 65 mm, having a typical range of 40 to 50 mm in length. Of course, one skilled in the art would appreciate that the diameter and length of the anchoring mechanism(s) and the screw or bolt may vary depending on the application and structural tissue to be applied to, repaired or reconstructed.

Of course, a physician or one skilled in the art may be able to determine which coil 2360 and screw (bolt, etc.) dimension combination may be appropriately used with one another based on the wear pattern of the pedicle channel as shown in FIGS. 22A, 22B, and 22C. For example, if the pedicle channel is worn such that the inner portion of the pedicle channel is formed or worn more than the opening, then it may be more appropriate to use a coil 2360 and screw (bolt, etc.) 2370 combination which will cause the bottom portion of the coil 2360 to expand to fill the more severely worn portion of the worn pedicle channel while minimizing expansion near the opening of the worn pedicle channel. If the pedicle channel is formed or worn such that the opening of the pedicle channel is more severely worn than the inner portion, then it may be more appropriate to use a coil 2360 and screw (bolt, etc.) 2370 combination which will cause the upper portion of the coil 2360 to expand to fill the more widely formed or worn portion of the channel while minimizing expansion near the inner portion of the formed or worn channel.

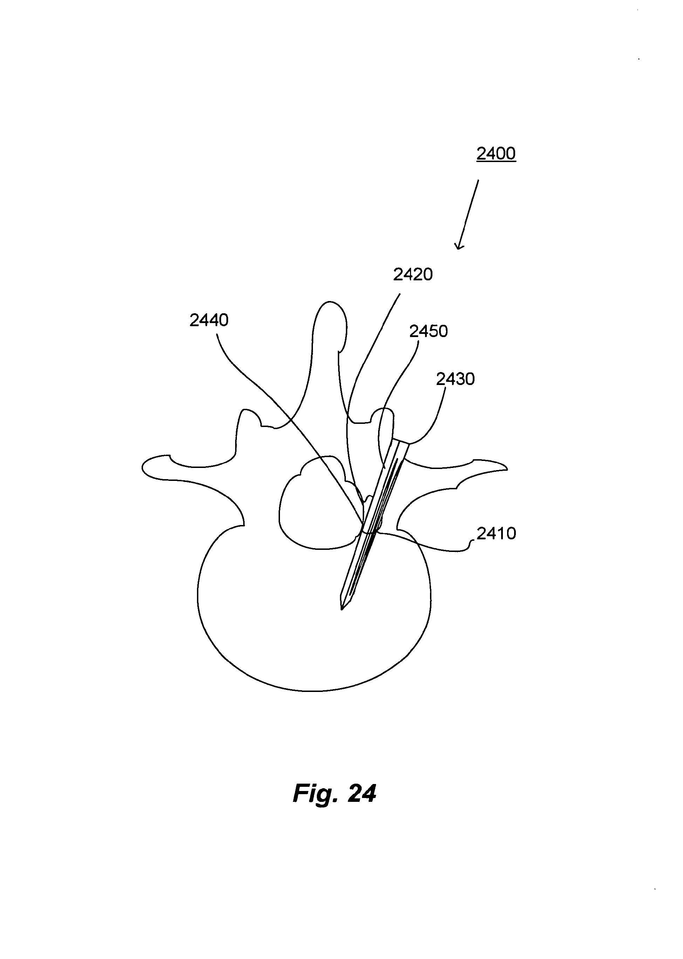

Referring now to Fig. 24, a top view of a vertebra 2400 is shown having a pedicle channel anchoring hole 2410 that has breached the cortical pedicle wall 2420. One exemplary embodiment of the anchoring mechanism 2430 may contain a side that may smooth 2450 or/and an end 2440 that may be covered with a protective coating. The anchoring mechanism 2430 may be inserted into the worn pedicle channel anchoring hole 2410 such that the smooth side 2450 or/and end 2440 with the protective coating are facing the breached cortical pedicle wall 2420. The smooth surface side 2450 or/and protective coating 2440 may reduce possible irritation of or prevent spinal cord or nerve tissue from becoming irritated by the breached cortical pedicle wall 2420 or/and the anchoring mechanism 2430 inserted into the worn pedicle channel anchoring hole 2410.

Referring now to Figs. 25 - 29, various means of holding and positioning an anchoring mechanism in the channel in structural tissue into which it is placed while inserting or turning a screw, so as to prevent turning and improve aiming orientation of the anchoring mechanism, are presented. The various types of anchoring mechanisms of the present invention may add the ability to improve gripping or purchase of a screw or bolt, but placement and holding of the anchoring mechanism may be difficult. This may be particularly true when the structural tissue channel is of an irregular shape or larger than the anchoring mechanism, and/or when the screw or bolt is being turned into the anchoring mechanism. In Fig. 25, a perspective view 2500 of one exemplary embodiment of the anchoring mechanism 2530 is provided. A holder 2510 may be integrally formed to the anchoring mechanism 2530 or attached by, for example, a semi-permanent means to the anchoring mechanism 2530 for aligning and gripping during the insertion of a screw (bolt, etc.) into the opening 2550 of the anchoring mechanism 2530. The holder 2510 may be, for example, attached to the rim 2570 of the anchoring mechanism 2530 by smaller portions, e.g., 2520, so that the holder 2510 may be later removed by, for example, snapping off or cutting to disconnect it from the anchoring mechanism 2530. The anchoring mechanism 2500 may be placed into the pedicle channel. The handle 2560 of the holder 2510 may be gripped during the insertion of the screw (bolt, etc.) into the opening 2550 of the anchoring mechanism 2500 in order to minimize the minimize or eliminate any stress or torsion being translated on the surrounding structural tissue and to stabilize the anchoring mechanism 2530. The holder 2510 may also be used to adjust the positioning and angle of the anchoring mechanism 2530. Once the insertion of the screw (bolt, etc.) is complete, the holder 2510 may be removed by, for example, snapping or cut it from the anchoring mechanism 2530.

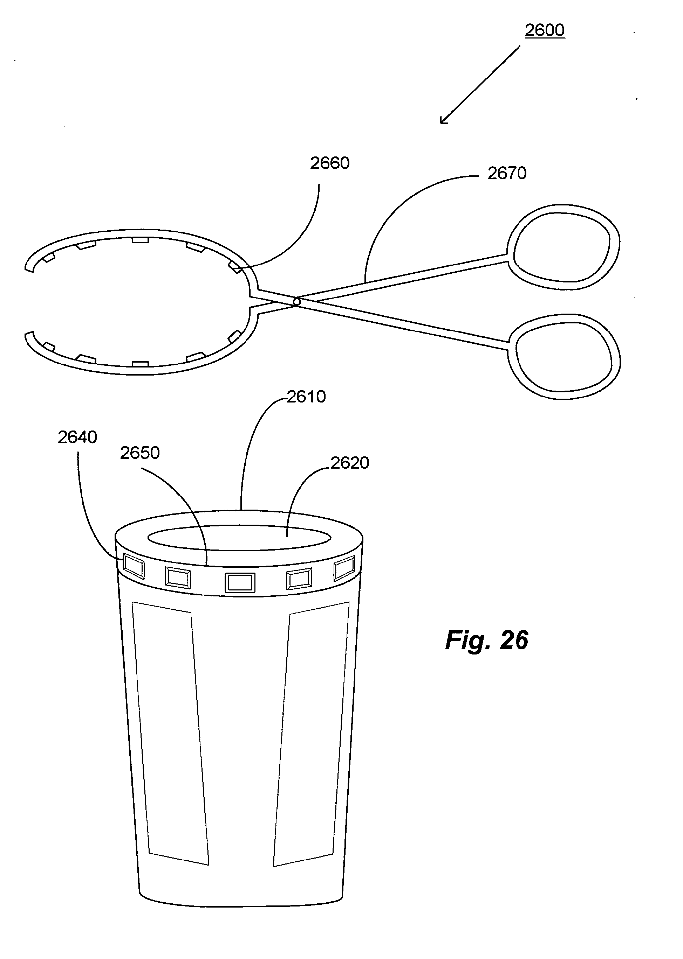

Referring now to Fig. 26, shows a perspective view 2600 of another exemplary embodiment of the anchoring mechanism 2610 along with a top view of customized forceps 2670. The rim 2650 of the anchoring mechanism 2610 may be lined with indentations or holes, e.g., 2640, to aid in gripping and holding the anchoring mechanism 2610 to provide control and eliminate turning or movement of the anchoring mechanism 2610 when the screw (bolt, etc.) is inserted into the opening 2620 and screwed or pushed into the lateral length anchoring mechanism 2610. The forceps 2670 may be constructed to match the shape of the rim 2650 and include posts or teeth 2660 that may be constructed to align with and match the location and shape of the indentations or holes 2640 along the circumference of the anchoring mechanism 2610. The anchoring mechanism 2610 may be inserted into a pedicle channel by hand (e.g., if a screw is partially inserted in the anchoring mechanism), with a pair of pliers, and/or with the forceps 2670. A screw (bolt, etc.) may then be inserted into the opening 2620 of the anchoring mechanism 2610 while using the forceps 2670 to grip the rim 2650 of the anchoring mechanism 2610. The forceps 2670 used with the rim 2650 of the anchoring mechanism 2610 may be used to adjust the positioning and angle of the anchoring mechanism 2610. Gripping the rim 2650 may help minimize the effect of stress and/or torsion on the surrounding structural tissue and stabilize the anchoring mechanism 2610.

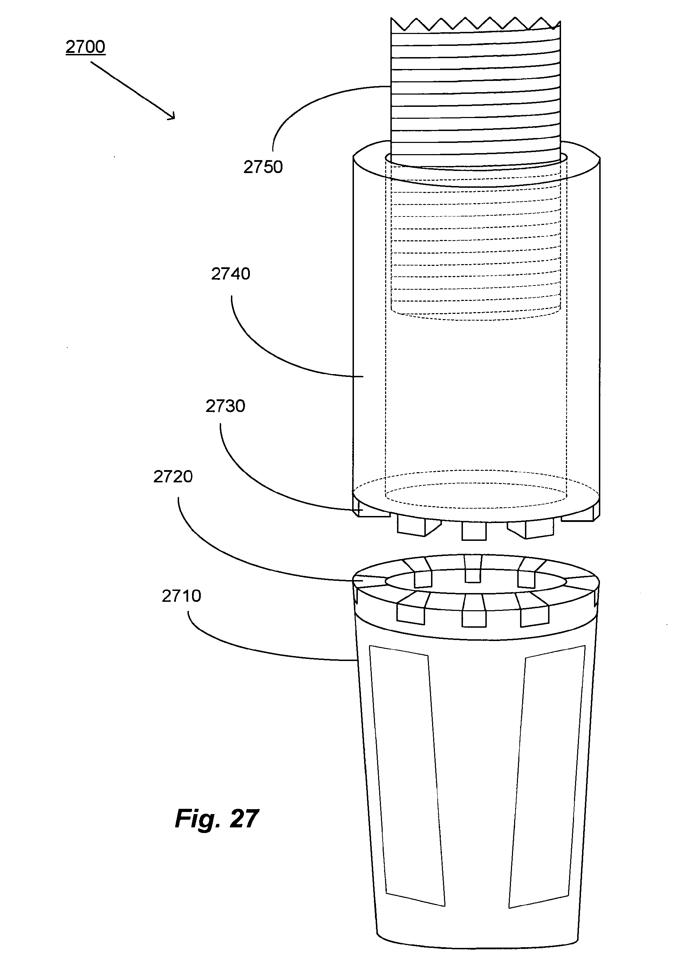

Referring now to Fig. 27, a perspective view of one exemplary embodiment of the anchoring mechanism 2710 with a slotted rim 2720 are provided. The slotted rim 2720 may be constructed to fit teeth 2730 of a cylindrical guide 2740. The cylindrical guide 2740 may be positioned to match and be interdigitated with the slotted rim 2720 when place on top of the anchoring mechanism 2710. The cylindrical guide 2740 may be designed with a allow center for proper positioning of the screw (bolt, etc.) 2750 to enable ease of insertion into the anchoring mechanism 2710 while providing a means to grip the cylindrical guide 2740, which in turn grips the slotted rim 2720 of the anchoring mechanism 2710. This configuration may be used to minimize turning or torsion during the insertion of the screw (bolt, etc.) 2750. Proper positioning of the screw (bolt, etc.) 2750 may be more easily accomplished once the cylindrical guide 2740 is placed and utilized, since one skilled in the art may use the cylindrical guide 2740 to lead the screw (bolt, etc.) 2750 directly to the point of insertion on the anchoring mechanism 2710. Gripping the rim 2720 of the cylindrical guide 2740 during insertion of the screw (bolt, etc.) may also help minimize the effect of torsion on the surrounding tissue. Although the teeth 2730 of the cylindrical guide and holder and the corresponding grooves or slots in the rim 2720 are shown a simple rectangular shapes, they may be a more complicated locking shape, such as an "L" shape that will allow connecting and disconnecting with vertical control and circular stopping.

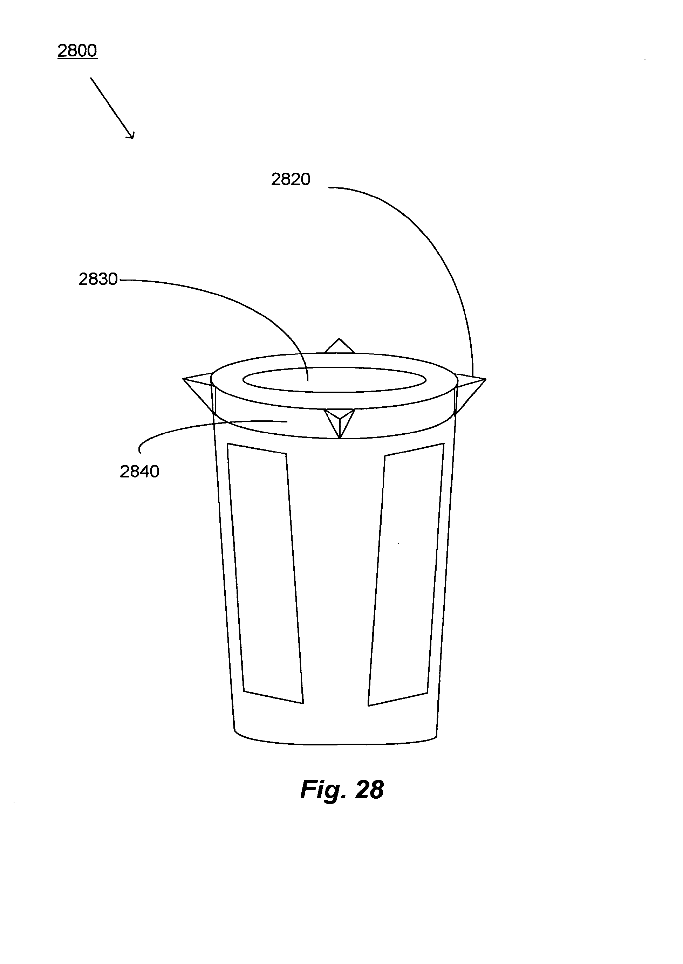

Referring now to Fig. 28, a perspective view of an exemplary embodiment of the anchoring mechanism 2800 with a rim 2840 containing gripping members 2820 that extend laterally outward from the anchoring mechanism 2800 is shown. The gripping members 2820 may be triangular and tapered to aid in the insertion of the anchoring mechanism 2800 into the pedicle and gripping into an outer structural tissue surface. During the insertion of a screw (bolt, etc.) into the opening 2830 of the anchoring mechanism 2800 and after the anchoring mechanism has been inserted into the pedicle, the gripping members 2820 may provide a force to counteract the torsion or circular motion of the screw (bolt, etc.) as it is being turned into the anchoring mechanism 2800, so as to minimize the circular movement of the anchoring mechanism 2800 or effect of torsion on the surrounding structural tissue.

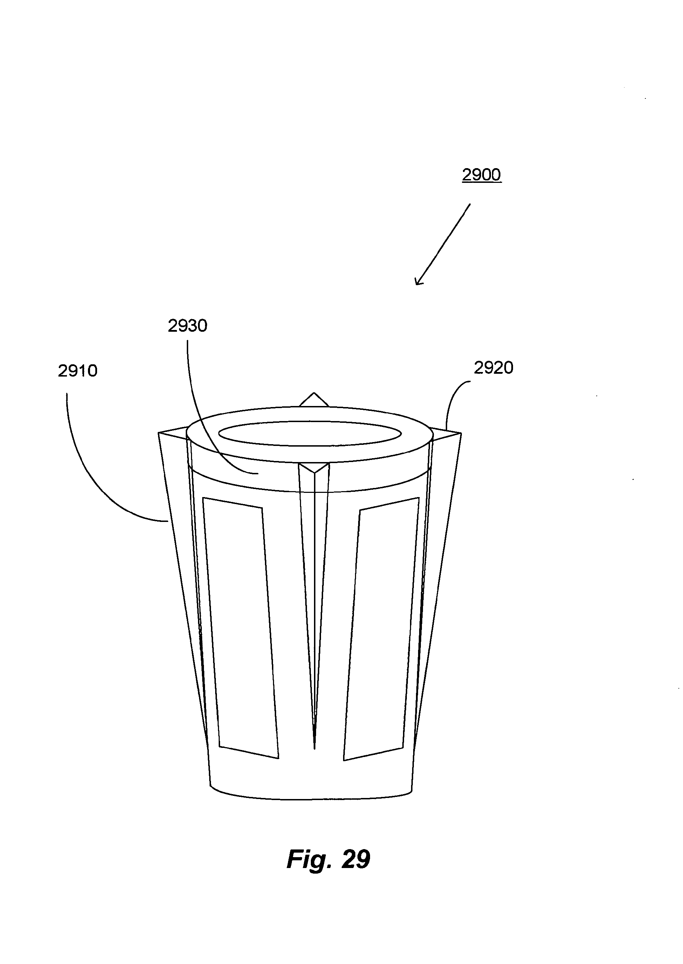

Referring now to Fig. 29, a perspective view depicts an exemplary embodiment of the anchoring mechanism 2900 with a rim 2930 that may contain gripping members 2920 that may extend along the lateral length of the anchoring mechanism 2900. In this embodiment, the gripping members 2920 may be tapered 2910 and may span most of the entire lateral length of the anchoring mechanism 2900. In one variation, the gripping members 2920 may be eliminated from the rim 2930 and be formed only on the side walls of the anchoring mechanism 2900. In any case, the span of tapering 2910 and length of gripping members 2920 may provide more force to counteract the torsion of the screw (bolt, etc.) being inserted or screwed into the anchoring mechanism 2900 and may even provide further improved gripping or purchase between the anchoring mechanism 2900 and the surrounding structural tissue.

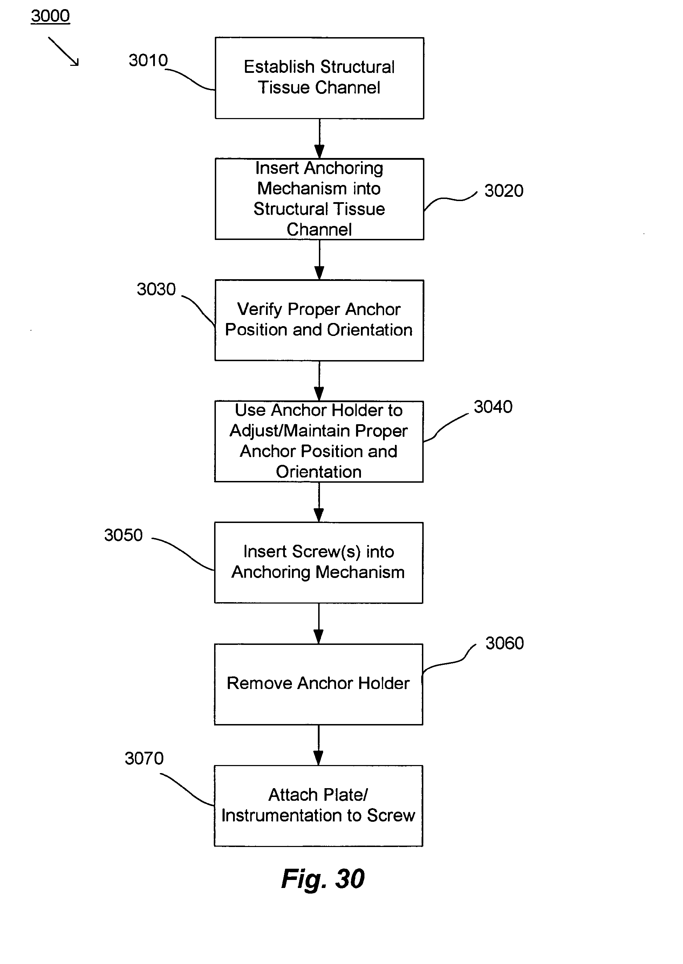

Referring now to Fig. 30, a block diagram 3000 showing a method of using the present invention is provided, according to one exemplary embodiment. First, at step 3010, a passage or channel is established or formed in a portion of structural tissue. A structural tissue channel may be established by any method currently or prospectively utilized, for example, using a drill and a drill bit or a pick styled device. However, one skilled in the art would recognize that the method of establishing a structural tissue channel may vary according to many factors such as the type of structural tissue, the location of the desired structural tissue channel, or other factors specific to the variance associated with different patients' anatomy or specific needs. Next, a step 3020 an anchoring mechanism may be inserted into the established structural tissue channel. The anchoring mechanism may be characterized by, but not limited to, the various embodiments depicted above in FIGS. 1 - 29 and/or may include expandable characteristics with one or more means of holding and orienting the anchoring mechanism. One skilled in the art would recognize that the insertion of the anchoring mechanism may vary according to factors specific to the variance associated with different patients' anatomy or specific types of structural tissue or needs as required to successfully complete the task. Then, at step 3030, proper anchoring mechanism positioning and orientation may be verified using, for example, visual cues and/or markers. For example, markers as depicted in Fig. 8 and described above may provide useful information regarding the position of the anchoring mechanism in the channel or pedicle including, but not limited to, the position of the anchoring mechanism in the channel or pedicle, the length and width of the anchoring mechanism once inside the channel or pedicle, the angle of entry into the channel or pedicle, and/or the depth of the anchoring mechanism in the channel or pedicle. The marker(s) depicted in FIG. 8 may enable a physician or one skilled in the art to visually identify or understand the rotational and/or tilt orientation of the anchoring mechanism once inserted into the pedicle, and may be performed before and/or after a screw is fully inserted into the anchoring mechanism. So, then at step 3040 an anchoring mechanism holder may be used to adjust and/or maintain proper anchoring mechanism position and orientation in the channel or pedicle. Although, proper positioning and orientation of the anchoring mechanism and use of an anchor holder may occur prior to step 3040, for example as early as step 3020. In any case, the anchor holder may be embodied, but is not limited to, as depicted in FIGS. 25-29. For example, the holder may be a pair of pliers or forceps (without posts or ribs) or a pair of pliers may be used to insert the anchoring mechanism in step 3020. In any case a holding means or holder may be used to stabilize the anchoring mechanism for insertion of the screw (bolt, etc.) and/or may also help minimize the effect of torsion on the surrounding tissue as the screw (bolt, etc.) is inserted according to step 3050. Then, in step 3050, the screw (bolt, etc.) may be inserted into the anchoring mechanism by, for example, turning the screw while the anchoring mechanism is held still in place with a holder or holding means. Next, in step 3060, the anchor holder may be removed if it is a temporary anchor holder used for maintaining proper anchor position and/or orientation until the screw has been inserted into the anchoring mechanism. Finally, according to step 3070, if needed, an number of items may be attached to, for example, the head of the screw. For example, a plate and/or other instrumentation may be attached to the anchored screw, such as instrumentation for spinal cord fusing or fixation.

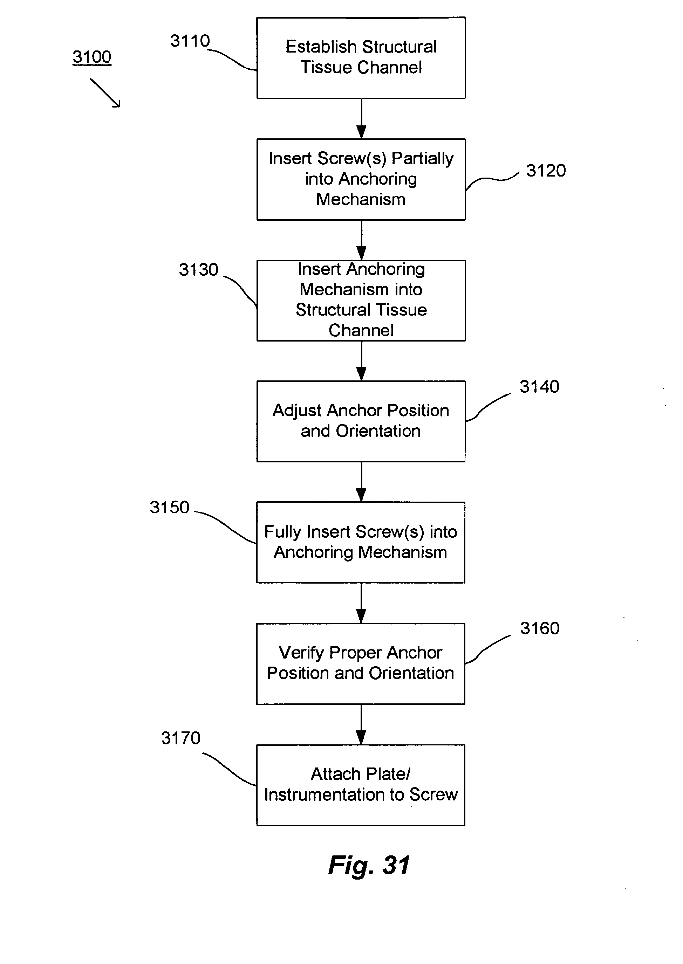

Referring now to Fig. 31, a block diagram showing another method of using the present invention is provided. Step 3110 involves establishing a passage or channel in a portion of structural tissue. A structural tissue channel may be established by any method currently or prospectively utilized. One skilled in the art would recognize that the method of establishing a structural tissue channel may vary according to many factors such as the type of structural tissue, the location of the desired structural tissue channel, or other factors specific to the variance associated with different patients' anatomy or specific needs. Step 3120 involves partially inserting the screw (bolt, etc.) into the anchoring mechanism. This step may be performed prior to the insertion of the anchoring mechanism into a structural tissue channel or pedicle channel. Step 3130 involves inserting an anchoring mechanism into the established structural tissue channel. The anchoring mechanism may be characterized by, but not limited to, the various embodiments depicted in FIGS. 1 - 29, and may include the use of a holding mechanism and/or pliers. One skilled in the art would recognize that the insertion of the anchoring mechanism may vary according to factors specific to the variance associated with different patients' anatomy, the operation being performed, specific needs, etc. Step 3140 involves adjusting the anchor position and orientation as needed. Again, this may be performed using a holding mechanism, pliers, forceps, etc. aided by markers and vision equipment. Once the anchoring mechanism is inserted into the structural tissue and the initial proper position and orientation of the anchoring mechanism has been adjusted, step 3150 involves fully inserting the screw (bolt, etc.) into the anchoring mechanism. Step 3160 involves verifying proper anchor and/or anchoring mechanism position and orientation using for example visual cues. The visual cues may include markers such as visual markers, x-ray opaque or sound identifiable markers, etc., depicted in FIG. 8 and/or described above. Use of markers or visual cues may provide useful information regarding the position of the anchoring mechanism in the structural tissue channel or pedicle including, but not limited to, the position of the anchoring mechanism in the channel or pedicle, the length and width of the anchoring mechanism once inside the channel or pedicle, the angle of entry into the channel or pedicle, or the depth of the anchoring mechanism in the channel or pedicle. The markers depicted in FIG. 8 may further allow one skilled in the art to visualize the rotational orientation of the anchoring mechanism during and after it is inserted into the channel or pedicle. According to step 3170, one skilled in the art may then attach the various instrumentation or plate to the anchored screw.

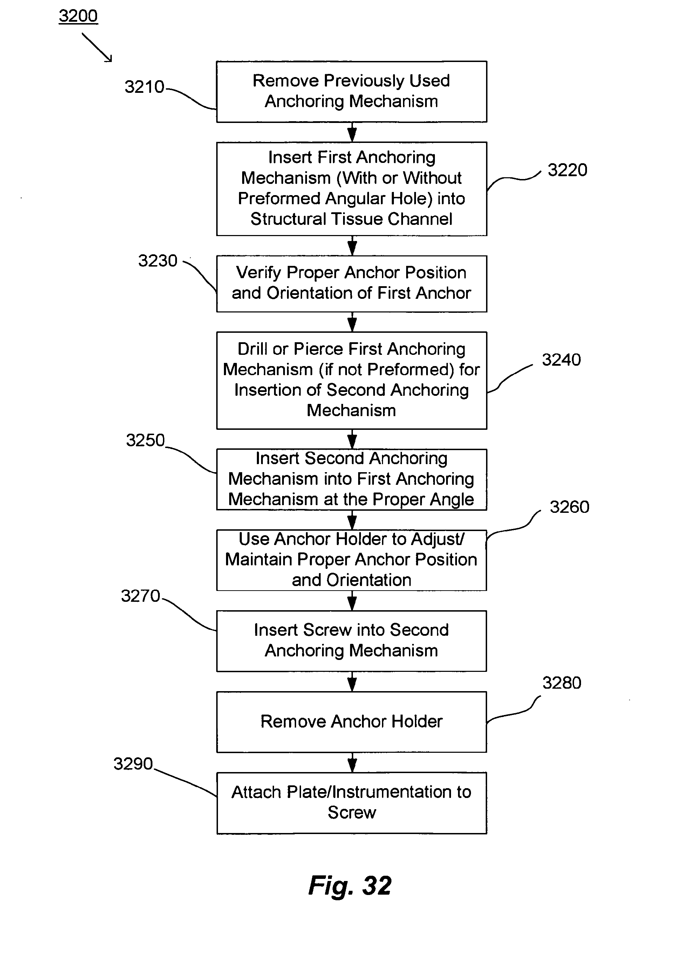

Referring to Fig. 32, a block diagram is provided showing a still further method of using the present invention which may involve using multiple anchoring mechanisms to redirect screw orientation or alignment and/or improve gripping or purchase. Step 3210 may involve removing a previously used anchoring mechanism and/or pedicle screw or bone screw, which may no longer be functioning properly due to, for example, wear in the pedicle channel. Wear in the pedicle channel may have caused the previously used anchoring mechanism and/or pedicle screw or bone screw to become insecure, move, and/or become detached or worn out to the extent that the originally intended angle of the pedicle channel and the needed gripping to the channel is no longer maintained. Step 3220 may involve inserting the first anchoring mechanism into the previously used structural tissue channel. The anchoring mechanism may, or may not, have a preformed angular insertion hole designed to realign an anchoring. The anchoring mechanism used may utilize, but is not limited to, the design in one or more of the embodiments depicted in, for example, FIGS. 1 - 29. The first inserted anchoring mechanism may include an additional hole formed therein for receiving a second anchoring mechanism. The additional hole may be preformed, formed by, for example, drilling and/or a punch, and may be formed before or after the first anchoring mechanism is inserted to a structural tissue channel. One purpose of the first anchoring mechanism may be to provide purchase in a structural tissue channel that has become worn and/or improperly angled. A second purpose of the first anchoring mechanism may be to provide a secure structure for insertion of a second anchoring mechanism. Step 3230 may involve verifying proper anchor position and orientation of the first anchoring mechanism. Verification may include positioning of the first anchoring mechanism to fill the path and increase purchase in the worn pedicle channel, and also ensuring the first anchoring mechanism is properly positioned for the insertion of a second anchoring mechanism into the first anchoring mechanism. Step 3240 may involve tapping or drilling the first inserted anchoring mechanism for insertion of the second anchoring mechanism, if it was not preformed or formed prior to inserting the anchoring mechanism into the anchoring hole in structural tissue. As noted above, this step may be done either before or after the first anchoring mechanism is inserted into a structural tissue channel. Step 3250 may involve inserting the second anchoring mechanism into the first anchoring mechanism at the appropriate or desired angle. The first anchoring mechanism may be used to provide a secure structure for insertion of the second anchoring mechanism. Since the second anchoring mechanism may be inserted at an angle (e.g., an oblique angle) relative to the main lateral axis and opening of the first anchoring mechanism, it need not be inserted directly into the opening of the first anchoring mechanism. Use of an oblique or direct insertion of the second anchoring mechanism into the first anchoring mechanism should allow one skilled in the art to establish proper positioning and orientation of the second anchoring mechanism for insertion of a screw (bolt, etc.) for use with, for example, various types of purposes or instrumentation. Step 3260 may involve using the anchor holder to adjust and/or maintain proper anchor positioning and orientation. The anchor holder may be embodied, but not limited to, as depicted in FIGS. 25-29. As noted above, the anchor holder may be utilized in step 3220. In any case, the use of the holder may stabilize the anchoring mechanism for insertion of the screw (bolt, etc.) or may also help minimize the effect of torsion on the surrounding tissue as the screw (bolt, etc.) is inserted according to step seven 3270. Step 3270 may involve inserting a screw (bolt, etc.) into the second anchoring mechanism for use with, for example, various types of structural tissue repair (e.g., bone repair) instrumentation such as spinal plate(s) and/or vertebra fixation instrumentation. Step 3280 may involve removing the anchor holder if one was used. Step 3290 may involve attaching any desired, necessary, or appropriate structural tissue repair instrumentation, for example, spinal plate(s) and/or vertebra fixation instrumentation to a portion of the screw (bolt, etc.) such as the head of the screw.

While embodiments of the invention have been described above, it is evident that many alternatives, modifications and variations will be apparent to those skilled in the art. For example, in at least one embodiment of the anchoring mechanism(s), the anchoring mechanism(s) may be constructed from a biomaterial such as bone tissue generated, manufactured, and/or grown by, for example, a stem cell based process. The stem cells may be provided from cells of the patient, other people, fetus cells, or synthetically according to various known or developing techniques. The use of such a biogenerated and/or grown material may allow the screw (bolt, etc.) to be secured in the pedicle without some of the typical difficulties associated with introducing foreign materials into the human body. Accordingly, the embodiments of the invention, as set forth above, are intended to be illustrative, and should not be construed as limitations on the scope of the invention. Various changes may be made without departing from the spirit and scope of the invention. Accordingly, the scope of the present invention should be determined not by the embodiments illustrated above, but by the claims appended hereto and their legal equivalents.

## Claims

1. A structural tissue repair or reconstruction apparatus, comprising:
a cylindrically or conical shaped anchor mechanism including:
a primary circular wall with an inner circular surface defining an inner channel for accepting a shaft, screw, or bolt, and an outer circular surface forming a lateral length body, the primary and a first open end and a second open end at opposing ends of the lateral length;
one or more expandable wall member(s) that are formed along the lateral length body and are planner with the outer circular surface and extend into the inner channel when the expandable wall member(s) are in their unexpanded position, and are approximately planner to the inner circular surface and expand outward from an outer circular surface when in their expanded position upon insertion of a shaft, screw or bolt into the anchoring mechanism, so as to increase screw purchase in structural tissue when the expandable wall member(s) are in the expanded position;
wherein the lateral length body is made of a material different than that of the expandable wall members.

2. The repair or reconstruction apparatus of claim 1, wherein the lateral length body is made of a rigid material and the expandable wall members are made of a malleable material attached to the lateral length body.

3. The repair or reconstruction apparatus of claim 1, wherein at least one of the first open end or the second open end includes a rim attached thereto.

4. The repair or reconstruction apparatus of claim 1, wherein the expandable wall members is thicker at a center portion along the lateral length of the anchoring mechanism.

5. The repair or reconstruction apparatus of claim 1, wherein the expandable wall members is thicker at a location closest to the first open end or the second open end along the lateral length of the anchoring mechanism.

6. The repair or reconstruction apparatus of claim 1, wherein the outer circular surface(s) of the anchoring mechanism has at least a portion that is textured, threaded, ribbed, or studded.

7. The repair or reconstruction apparatus of claim 1, wherein the outer circular surface(s) of the anchoring mechanism is composed of a gripping material made of allograft, allograft bone, polyetheretherkeetones (PEEK), high molecular weight polyethylene (HMWPE), carbon fiber, or other biocompatible material.

8. The repair or reconstruction apparatus of claim 1, wherein the outer circular surface(s) of the anchoring mechanism is coated with hydroxyl apatite or bone morphogenetic protein.

9. The repair or reconstruction apparatus of claim 1, further comprising:
a means for holding and positioning the anchoring mechanism in a channel in structural tissue.

10. The repair or reconstruction apparatus of claim 1, further comprising:
a marker means for visually determining position and orientation of the anchoring mechanism.

11. An anchoring system, comprising:
a first anchoring mechanism;
a second anchoring mechanism; and
a rod, screw or bolt extending through at least a portion of the first anchoring mechanism and the second anchoring mechanism.

12. The anchoring system of claim 11, wherein the second anchoring mechanisms is inserted into the first anchoring.

13. The anchoring system of claim 12, wherein the second anchoring mechanisms is inserted into the first anchoring mechanism at an angle relative to the lateral axis of the first anchor.

14. An anchoring mechanism, comprising:
a means for gripping the anchoring mechanism to hold it in a desired position.

15. The anchoring mechanism of claim 14, wherein the means for gripping the anchoring mechanism is a handle formed as part of the anchoring mechanism that can be removed after the anchoring mechanism is placed into a desired location and a rod, screw or bolt is inserted to the anchoring mechanism.

16. The anchoring mechanism of claim 14, wherein an external circumference near one end of the anchoring mechanism includes holes, is slotted, indented, or is ribbed for gripping the anchoring mechanism.

17. The anchoring mechanism of claim 14, wherein a guide to hold the anchoring mechanism is coupled to the anchoring mechanism via a plurality of slots in the external circumference.

18. The anchoring mechanism of claim 17, wherein the guide includes a rim with indentations interdigitated with the rim of the anchoring mechanism.

19. An anchoring mechanism utilized in a medical treatment(s), comprising:
an anchor with an external surface that contains members that extend laterally outward to counter the rotational forces caused by threading a screw or bolt into the anchor.

20. The anchoring mechanism of claim 19, wherein the members of the external surface of the anchor extend lengthwise across the entire surface of the anchor.
